(19) Europäisches Patentamt
European Patent Office
Office européen des brevets

(11) **EP 1 920 597 B1**

(12) **EUROPEAN PATENT SPECIFICATION**

(45) Date of publication and mention
of the grant of the patent:
**22.09.2021 Bulletin 2021/38**

(21) Application number: **06813840.3**

(22) Date of filing: **28.08.2006**

(51) Int Cl.:
*G06K 9/00* (2006.01)        *G06K 9/62* (2006.01)
*G06K 9/20* (2006.01)        *H01L 27/146* (2006.01)
*H01L 31/0232* (2014.01)     *H04N 9/04* (2006.01)
*H04N 5/335* (2011.01)       *A61B 5/00* (2006.01)

(86) International application number:
**PCT/US2006/033485**

(87) International publication number:
**WO 2007/027579 (08.03.2007 Gazette 2007/10)**

(54) **BIOMETRIC SENSORS**

BIOMETRISCHE SENSOREN

CAPTEURS BIOMETRIQUES

(84) Designated Contracting States:
**AT BE BG CH CY CZ DE DK EE ES FI FR GB GR
HU IE IS IT LI LT LU LV MC NL PL PT RO SE SI
SK TR**

(30) Priority:   **01.09.2005  US 21900605
19.07.2006  US 458619
19.07.2006  US 458607**

(43) Date of publication of application:
**14.05.2008 Bulletin 2008/20**

(73) Proprietor: **HID Global Corporation
Austin, TX 78753 (US)**

(72) Inventors:
• **ROWE, Robert, K.**
**Corrales, New Mexico 87048 (US)**
• **CORCORAN, Stephen, P.**
**Corrales, New Mexico 87048 (US)**
• **NIXON, Kristin, A.**
**Albuquerque, New Mexico 87111 (US)**
• **DOUCET, Todd**
**Albuquerque, New Mexico 87111 (US)**
• **MARTIN, Ryan**
**Albuquerque, New Mexico 87112 (US)**

(74) Representative: **DTS Patent- und Rechtsanwälte
Schnekenbühl und Partner mbB
Marstallstrasse 8
80539 München (DE)**

(56) References cited:
**WO-A2-2004/090786        WO-A2-2005/059805
US-A- 5 291 560           US-A1- 2002 183 624
US-A1- 2004 125 994       US-A1- 2004 125 994
US-B1- 6 292 576          US-B1- 6 292 576**

• **RANDEN T ET AL: "Filtering for texture
classification: a comparative study", IEEE
TRANSACTIONS ON PATTERN ANALYSIS AND
MACHINE INTELLIGENCE, IEEE COMPUTER
SOCIETY, USA, vol. 21, no. 4, 1 April 1999
(1999-04-01), pages 291-310, XP002336483, ISSN:
0162-8828, DOI: 10.1109/34.761261**

**Description**

BACKGROUND OF THE INVENTION

This application relates generally to biometrics. More specifically, this application relates to methods and systems for performing biometric measurements that use spectral information.

"Biometrics" refers generally to the statistical analysis of characteristics of living bodies. One category of biometrics includes "biometric identification," which commonly operates under one of two modes to provide automatic identification of people or to verify purported identities of people. Biometric sensing technologies measure the physical features or behavioral characteristics of a person and compare those features to similar prerecorded measurements to determine whether there is a match. Physical features that are commonly used for biometric identification include faces, irises, hand geometry, vein structure, and fingerprint patterns, which is the most prevalent of all biometric-identification features. Current methods for analyzing collected fingerprints include optical, capacitive, radio-frequency, thermal, ultrasonic, and several other less common techniques.

In WO 2005/ 059805A2 the applicant describes methods and sytsems for estimation of personal characteristics from biometric measurements. In WO 2004/ 090786A2 the applicant describes a multispectral biometric sensor. In US 2002/183624A1 the applicant describes an apparatus and a method of biometric determination using specialized optical spectroscopy systems.

Most of the fingerprint-collection methods rely on measuring characteristics of the skin at or very near the surface of a finger. In particular, optical fingerprint readers typically rely on the presence or absence of a difference in the index of refraction between the sensor platen and the finger placed on it. When an air-filled valley of the fingerprint is above a particular location of the platen, total internal reflectance ("TIR") occurs in the platen because of the air-platen index difference. Alternatively, if skin of the proper index of refraction is in optical contact with the platen, then the TIR at this location is "frustrated," allowing light to traverse the platen-skin interface. A map of the differences in TIR across the region where the finger is touching the platen forms the basis for a conventional optical fingerprint reading. There are a number of optical arrangements used to detect this variation of the optical interface in both bright-field and dark-field optical arrangements. Commonly, a single, quasimonochromatic beam of light is used to perform this TIR-based measurement.

There also exists non-TIR optical fingerprint sensors, bi most cases, these sensors rely on some arrangement of quasimonochromatic light to illuminate the front, sides, or back of a fingertip, causing the light to diffuse through the skin. The fingerprint image is formed due to the differences in light transmission across the skin-platen boundary for the ridge and valleys. The difference in optical transmission are due to changes in the Fresnel reflection characteristics due to the presence or absence of any intermediate air gap in the valleys, as known to one of familiarity in the art.

Optical fingerprint readers are particularly susceptible to image quality problems due to non-ideal conditions. If the skin is overly dry, the index match with the platen will be compromised, resulting in poor image contrast. Similarly, if the finger is very wet, the valleys may fill with water, causing an optical coupling to occur all across the fingerprint region and greatly reducing image contrast. Similar effects may occur if the pressure of the finger on the platen is too little or too great, the skin or sensor is dirty, the skin is aged and/or worn, or overly fine features are present such as may be the case for certain ethnic groups and in very young children. These effects decrease image quality and thereby decrease the overall performance of the fingerprint sensor, bi some cases, commercial optical fingerprint readers incorporate a thin membrane of soft material such as silicone to help mitigate these effects and restore performance. As a soft material, the membrane is subject to damage, wear, and contamination, limiting the use of the sensor without maintenance.

Optical fingerprint readers, such as those based on TIR, as well as other modalities such as capacitance, RF, and others, typically produce images that are affected to some degree by the nonideal imaging conditions present during acquisition. An analysis of the textural characteristics of the resulting images is thus affected by the sampling conditions, which may. limit or obscure the ability to observe the textural characteristics of the person's skin. The consequence of this is that texture is of limited utility in such sensing modalities.

Biometric sensors, particularly fingerprint biometric sensors, are generally prone to being defeated by various forms of spoof samples, hi the case of fingerprint readers, a variety of methods are known in the art for presenting readers with a fingerprint pattern of an authorized user that is embedded in some kind of inanimate material such as paper, gelatin, epoxy, latex, and the like. Thus, even if a fingerprint reader can be considered to reliably determine the presence or absence of a matching fingerprint pattern, it is also critical to the overall system security to ensure that the matching pattern is being acquired from a genuine, living finger, which may be difficult to ascertain with many common sensors.

Another way in which some biometric systems may be defeated is through the use of a replay attack, hi this scenario, an intruder records the signals coming from the sensor when an authorized user is using the system. At a later time, the intruder manipulates the sensor system such that the prerecorded authorized signals may be injected into the system, thereby bypassing the sensor itself and gaining access to the system secured by the biometric.

A common approach to making biometric sensors more robust, more secure, and less error-prone is to combine sources of biometric signals using an approach sometimes referred to in the art as using "dual," "combinatoric," "layered," "fused," "multibiometric," or "multifactor biometric" sensing. To provide enhanced security in this way, biometric technologies are combined in such a way that different technologies measure portions of the body at the same time and are resistant to being defeated by using different samples or techniques to defeat the different sensors that are combined. When technologies are combined in a way that they view the same part of the body they are referred to as being "tightly coupled."

The accuracy of noninvasive optical measurements of physiological analytes such as glucose, alcohol, hemoglobin, urea, and cholesterol can be adversely affected by variation of the skin tissue, hi some cases it is advantageous to measure one or more physiological analytes in conjunction with a biometric measurement. Such dual measurement has potential interest and application to both commercial and law-enforcement markets.

There is accordingly a general need in the art for improved methods and systems for biometric sensing and analyte estimation using multispectral imaging systems and methods.

BRIEF SUMMARY OF THE INVENTION

The object of the invention is achieved by a an apparatus according to claim 1. Further embodiments are defined in the dependent claims.

The sample is illuminated under a plurality of distinct optical conditions. Light scattered from the sample is received. A plurality of images are formed, each such image being formed from the received light for a respective one of the plurality of distinct optical conditions. A plurality of texture measures is generated, each such texture measure being generated from a respective one of the plurality of images. It is determined whether the generated plurality of texture measures is consistent with the sample being authentic unconcealed biological tissue. The texture measure may comprise an image-contrast measure is certain specific embodiments.

Illumination of the sample under distinct optical conditions may be achieved by illuminating it with light having a plurality of different wavelengths, illuminating it with light under a plurality of distinct polarization conditions, illuminating it with light at a plurality of distinct illumination angles, and the like.

The texture measure may be generated in some embodiments by performing a spatial moving-window analysis of each of the plurality of images, in some instances, the plurality of texture measures may comprise a measure of image contrast within certain spatial frequencies. For example, when the sample is illuminated with light having a plurality of distinct wavelengths, it may be confirmed that an image contrast under red illumination is less than an image contrast under blue illumination. Alternatively, it may be confirmed that an image contrast under red illumination is less than a predetermined value. The spatial moving-window analysis may be performed in some instances by calculating moving window Fourier transforms on the plurality of images. Alternatively, it may be performed by calculating a moving-window centrality and a moving-window variability measure of the plurality of images. For instance, in one embodiment, the moving-window centrality measure comprises a moving-window mean and the moving-window variability measure comprises a moving-window standard deviation. In another embodiment, the moving-window Fourier transforms on the plurality of images. Alternatively, it may be performed by calculating a moving-window centrality and a moving-window variability measure of the plurality of images. For instance, in one embodiment, the moving-window centrality measure comprises a moving-window mean and the moving-window variability measure comprises a moving-window standard deviation. In another embodiment, the moving-window centrality measure comprises a moving-window mean and the moving-window variability measure comprises a moving-window range.

The determination of sample authenticity may be made by applying a multidimensional scaling to map the plurality of texture measures to a point in a multidimensional space. This permits a determination to be made whether the point is in a predefined region of the multidimensional space that corresponds to the sample being authentic unconcealed biological tissue. In one embodiment, the multidimensional space is a two-dimensional space.

In another set of embodiments, a method is provided of performing a biometric function. A purported skin site of an individual is illuminated with illumination light. The purported skin site is in contact with a surface. Light scattered from the purported skin site is received substantially in a plane that includes the surface. An image is formed from the received light. An image-texture measure is generated from the image. The generated image-texture measure is analyzed to perform the biometric function.

In certain embodiments, the biometric function comprises an antispoofing function; in such embodiments the image-texture measure is analyzed to determine whether the purported skin site comprises living tissue. In other embodiments, the biometric function comprises an identity function; in such embodiments, the image-texture measure is analyzed to determine an identity of the individual. In still other embodiments, the biometric function comprises a demographic or anthropometric function; in such embodiments, the image-texture measure is analyzed to estimate a demographic or anthropometric characteristic of the individual.

The surface may be the surface of an imaging detector, with light scattered from the purported skin site being received at the imaging detector. Alternatively, a pattern of light may be translated from the plane to an imaging detector disposed outside the plane without substantial degradation or attenuation of the pattern, with the translated pattern being received at the imaging detector. In different embodiments, the light scattered from the purported skin site may be received at a monochromatic imaging detector or at a color imaging detector.

In some embodiments, the illumination light is white light. The image may then comprise a plurality of images corresponding to different wavelengths. The image-texture measure may accordingly be generated by performing a spatial moving-window analysis of each of the plurality of images. For instance, moving-window Fourier transforms may be calculated on the plurality of images. Alternatively, a moving-window centrality measure and a moving-window variability measure of the plurality of images maybe calculated.

In analyzing the generated image-texture measure to perform the biometric function, the generated image-texture measure may be compared with a reference image-texture measure. In some cases, the reference image-texture measure was generated from a reference image formed from light scattered from a reference skin site with the purported skin site being substantially different from the reference skin site. In certain embodiments, spectral features of the received light are compared with reference spectral features in performing the biometric function.

In a further set of embodiments, a biometric sensor is provided. The sensor comprises a surface, an illumination subsystem, a detection subsystem, and a computational unit. The surface is adapted for contact with a purported skin site. The illumination subsystem is disposed to illuminate the purported skin site when the purported skin site is in contact with the surface. The detection subsystem is disposed to receive light scattered from the purported skin site, with the light being received substantially in a plane that includes the surface. The computational unit is interfaced with the detection subsystem and has instructions for forming an image from the received light. It also has instructions for generating an image-texture measure from the image and for analyzing the generated image-texture measure to perform the biometric function.

There are a number of different biometric functions that may be performed with such a sensor. In one embodiment, the biometric function comprises an antispoofing function, with the computational unit having instructions for determining whether the purported skin site comprises living tissue. In another embodiment, the biometric function comprises an identity function, with the computational unit having instructions for determining an identify of the individual from the generated image-texture measure. In still another embodiment, the biometric function comprises a demographic or anthropometric function, with the computational unit having instructions for estimating a demographic or anthropometric characteristic of the individual from the generated image-texture measure.

In some instances, the biometric sensor further comprises an imaging detector. In one such embodiment, the surface is a surface of the imaging detector, with the detection subsystem comprising the imaging detector and being configured to receive light scattered from the purported skin site at the imaging detector. In another such embodiment, the imaging detector is disposed outside the plane. An optical arrangement is configured to translate a pattern of light from the plane to the imaging detector without substantial degradation or attenuation of the pattern. The detection system comprises the imaging detector and is configured to receive the translated pattern at the imaging detector. The imaging detector may comprise a monochromatic imaging detector or a color imaging detector in different embodiments.

In some cases, the illumination subsystem is configured to illuminate the purported skin site with white light. The image may then comprise a plurality of images corresponding to different wavelengths, with the instructions for generating the image-texture measure comprise instructions for performing a spatial moving-window analysis of each of the plurality of images. For instance, there may be instructions for calculating moving-window Fourier transforms on the plurality of images in one embodiment, while another embodiment has instructions for calculating a moving-window centrality measure and a moving-window variability measure of the plurality of images.

In one embodiment, the instructions for analyzing the generated image-texture measure to perform the biometric function comprise instructions for comparing the generated image-texture measure with a reference image-texture measure. Such a reference image-texture measure may have been generated from a reference image formed from light scattered from a reference skin site, with the purported skin site being substantially different from the reference skin site. In a certain embodiment, the computational unit further has instructions for comparing spectral features of the received light with reference spectral features in performing the biometric function.

In still another set of embodiments, a biometric sensor is provided. A white-light illumination subsystem is disposed to illuminate a purported skin site of an individual with white light. A detection subsystem is disposed to receive light scattered from the purported skin site and comprises a color imager on which the received light is incident. A computational unit is interfaced with the detection subsystem. The computational unit has instructions for deriving a plurality of spatially distributed images of the purported skin site from the received light with the color imager. The plurality of spatially distributed images correspond to different volumes of illuminated tissue of the individual. The computational unit also has instructions for analyzing the plurality of spatially distributed images to perform a biometric function.

In one of these embodiments, the biometric function comprises an antispoofing function and the instructions for analyzing the plurality of spatially distributed images comprise instructions for determining whether the purported skin site comprises living tissue. In another of these embodiments, the instructions for analyzing the plurality of spatially distributed images to perform the biometric function comprise instructions for analyzing the plurality of spatially distributed images to estimate a demographic or anthropometric characteristic of the individual. In still another of these embodiments, the instructions for analyzing the plurality of spatially distributed images to perform the biometric function comprise instructions for analyzing the plurality of spatially distributed images to determine a concentration of an analyte in blood of the individual.

In some embodiments, the biometric sensor may further comprise a platen in contact with the purported skin site, with the white-light illumination subsystem being adapted to illuminate the purported skin site through the platen. In other embodiments, the white-light illumination subsystem may instead be adapted to illuminate the purported skin site when the skin site is not in physical contact with the biometric sensor.

The white light may be provided in different ways in different embodiments. For example, in one embodiment, the white-light illumination subsystem comprises a broadband source of white light. In another embodiment, the white-light illumination subsystem comprises a plurality of narrow-band light sources and an optical arrangement to combine light provided by the plurality of narrow-band light sources. The plurality of narrow-band light sources may provide light at wavelengths that correspond to each of a set of primary colors. In some cases, the purported skin site and an illumination region where the purported skin site is illuminated are in relative motion.

Some embodiments make use of polarization by including a first polarizing in the illumination system disposed to polarize the white light. The detection system then comprises a second polarizer disposed to encounter the received light. The first and second polarizers may be crossed relative to each other. In other embodiments, the first and second polarizers may be parallel. In some embodiments, the first polarizer may be omitted while retaining the second. in some embodiments, two or more of these polarization options may be combined in a single device. The detection system may also sometimes include an infrared filter disposed to encounter the received light before the received light is incident on the color imager.

In certain instances, the purported skin site is a volar surface of a finger or hand and the biometric function comprises a biometric identification. The instructions for analyzing the plurality of spatially distributed images comprise instructions for deriving a surface fingerprint or palmprint image of the purported skin site from the plurality of spatially distributed images. The surface fingerprint or palmprint image is then compared with a database of fingerprint or palmprint images to identify the individual. In other embodiment where the biometric function comprises a biometric identification, the instructions for analyzing the plurality of spatially distributed images instead comprise instructions for comparing the plurality of spatially distributed images with a database of multispectral images to identify the individual.

In yet a further set of embodiments, a method is provided of performing a biometric function. A purported skin site of an individual is illuminated with white light. Light scattered from the purported skin site is received with a color imager on which the received light is incident. A plurality of spatially distributed images of the purported skin site are derived, with the plurality of spatially distributed images corresponding to different volumes of illuminated tissue of the individual. The plurality of spatially distributed images are analyzed to perform the biometric function.

In some of these embodiments, the biometric function comprises an antispoofing function and analyzing the plurality of spatially distributed images comprises determining whether the purported skin site comprises living tissue. In other of these embodiments, the plurality of spatially distributed images are analyzed to estimate a demographic or anthropometric characteristic of the individual. In still different ones of these embodiments, the plurality of spatially distributed images are analyzed to determine a concentration of an analyte in blood of the individual.

The purported skin site may sometimes be illuminated by directing the white light through a platen in contact with the purported skin site. In some instances, the purported skin site may be illuminated with a broadband source of white light, while in other instances a plurality of narrow-band beams, perhaps corresponding to a set of primary colors, may be generated and combined. The purported skin site might sometimes be in relative motion with an illumination region where the purported skin site is illuminated.

In one embodiment the while light is polarized with a first polarization and the received light scattered from the purported skin site is polarized with a second polarization. The first and second polarizations may be substantially crossed relative to each other or may be substantially parallel to each other. The received light may sometimes be filtered at infrared wavelengths before the received light is incident on the color imager.

In some instances, the biometric function comprises a biometric identification. For instance, the purported skin site could be a volar surface of a finger or hand. Analysis of the plurality of spatially distributed images could then proceed by deriving a surface fingerprint or palmprint image of the purported skin site from the plurality of spatially distributed images and comparing the surface fingerprint or palmprint image with a database of fingerprint or palmprint images. In an alternative embodiment, the plurality of spatially distributed images could be compared with a database of multispectral images to identify the individual.

BRIEF DESCRIPTION OF THE DRAWINGS

A further understanding of the nature and advantages of the present invention may be realized by reference to the remaining portions of the specification and the drawings wherein like reference labels are used throughout the several drawings to refer to similar components. In some instances, reference labels include a numerical portion followed by a latin-letter suffix; reference to only the numerical portion of reference labels is intended to refer collectively to all reference labels that have that numerical portion but different latin-letter suffices.

Fig. 1 provides a front view of a noncontact biometric sensor in one embodiment of the invention;

Fig. 2A provides an illustration of a structure for a Bayer color filter array, which may be used in embodiments of the invention;

Fig. 2B is a graph showing color response curves for a Bayer color filter array like that illustrated in Fig. 2A;

Fig. 3 provides a front view of a noncontact biometric sensor in another embodiment of the invention;

Fig. 4 provides a top view of a sensor configuration that collects data during relative motion between a skin site and an optically active region of the sensor;

Fig. 5 illustrates a multispectral datacube that may be used in certain embodiments of the invention;

Fig. 6 is a front view of a contact biometric sensor in one embodiment of the invention;

Fig. 7A provides a side view of a contact biometric sensor in an embodiment;

Fig. 7B provides a side view of a contact biometric sensor in another embodiment;

Fig. 8 provides a front view of a contact biometric sensor in a further embodiment of the invention;

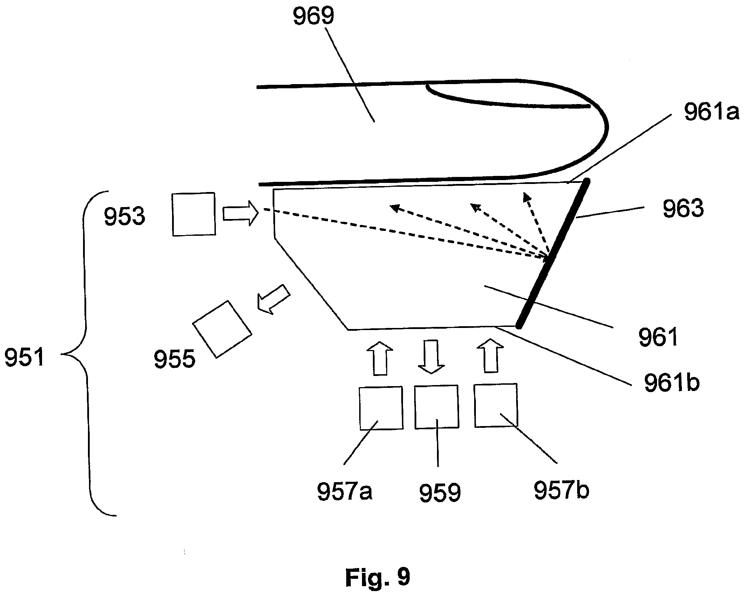

Fig. 9 provides a view of a multispectral biometric sensor in another embodiment of the invention;

Fig. 10A illustrates a structure for a contact texture biometric sensor in an embodiment of the invention;

Fig. 10B provides a side view of a contact texture biometric sensor in one configuration;

Fig. 10C provides a side view of a contact texture biometric sensor in another configuration;

Fig. 11 is schematic representation of a computer system that may be used to manage functionality of contact and noncontact biometric sensors in accordance with embodiments of the invention;

Fig. 12 is a graph of an oxygenated-blood absorbance spectrum;

Fig. 13 provides images taken under different illumination conditions of real fingers and of spoofs;

Fig. 14 is a flow diagram summarizing certain methods of the invention;

Fig. 15A provides a plot of results collected in a first embodiment of the invention;

Fig. 15B provides results comparing the data of Fig. 15A for real fingers and spoofs;

Fig. 15C is a separability plot resulting from multidimensional scaling of the data of Fig. 15A;

Fig. 16A provides a comparison of results collected in a second embodiment of the invention between real fingers and spoofs;

Fig. 16B is a separability plot resulting from multidimensional scaling of the data of Fig. 16A;

Fig. 17A provides a comparison of results collected in a third embodiment of the invention between real fingers and spoofs; and

Fig. 17B is a separability plot resulting from multidimensional scaling of the data of Fig. 17A;

Fig. 18 is a flow diagram summarizing methods of using contact and noncontact biometric sensors and illustrates a number of different biometric functions that may be performed; and

Fig. 19 is a flow diagram summarizing methods of operation of contact texture biometric sensors in accordance with embodiments of the invention.

DETAILED DESCRIPTION OF THE INVENTION

1. Overview

Embodiments of the invention provide methods and systems that allow for the collection and processing of a variety of different types of biometric measurements, including integrated, multifactor biometric measurements in some embodiments. These measurements may provide strong assurance of a person's identity, as well as of the authenticity of the biometric sample being taken. In some embodiments, a sensor uses white light that penetrates the surface of the person's skin, and scatters within the skin and/or the underlying tissue. As used herein, "white light" refers to light that has a spectral composition amenable to separation into constituent wavelength bands, which in some cases may comprise primary colors. The usual primary colors used to define white light are red, green, and blue, but other combinations may be used in other instances, as will be known to those of skill in the art. For clarity, it is emphasized that "white light" as used herein might not appear white to a human observer and might have a distinct tint or color associated with it because of the exact wavelength distribution and intensity of the constituent wavelength bands. In other cases, the white light may comprise one or more bands in the ultraviolet or infrared spectral regions. In some cases, the white light might not even be visible at all to a human observer when it consists of wavelength bands in the infrared and/or ultraviolet spectral regions. A portion of the light scattered by the skin and/or underlying tissue exits the skin and is used to form an image of the structure of the tissue at and below the surface of the skin. Because of the wavelength-dependent properties of the skin, the image formed from each wavelength of light comprised by the white light may be different from images formed at other wavelengths. Accordingly, embodiments of the invention collect images in such a way that characteristic spectral and spatial information may be extracted from the resulting image.

In some applications, it may be desirable to estimate other parameters and characteristics of a body, either independently or in combination with a biometric measurement. For example, in one specific such embodiment, an ability is provided to measure analyte levels of a person simultaneously with measurement of a fingerprint pattern. Applications to law enforcement may be found in embodiments where the measure analyte comprises a blood-alcohol level of the person; such embodiments also enable a variety of commercial applications that include restricting motor-vehicle access. In this way, the analyte measurement and the identity of the person on whom the measurement is made may be inextricably linked.

Skin composition and structure is very distinct, very complex, and varies from person to person. By performing optical measurements of the spatiospectral properties of skin and underlying tissue, a number of assessments may be made. For example, a biometric-identification function may be performed to identify or verify whose skin is being measured, a liveness function may be performed to assure that the sample being measured is live and viable skin and not another type of material, estimates may be made of a variety of physiological parameters such as age gender, ethnicity, and other demographic and anthropometric characteristics, and/or measurements may be made of the concentrations of various analytes and parameters including alcohol, glucose, degrees of blood perfusion and oxygenation, biliruben, cholesterol, urea, and the like.

The complex structure of skin may be used in different embodiments to tailor aspects of the methods and systems for particular functions. The outermost layer of skin, the epidermis, is supported by the underlying dermis and hypodermis. The epidermis itself may have five identified sublayers that include the stratum corneum, the stratum lucidum, the stratum granulosum, the stratum spinosum, and the stratum germinativum. Thus, for example, the skin below the top-most stratum corneum has some characteristics that relate to the surface topography, as well as some characteristics that change with depth into the skin. While the blood supply to skin exists in the dermal layer, the dermis has protrusions into the epidermis known as "dermal papillae," which bring the blood supply close to the surface via capillaries. In the volar surfaces of the fingers, this capillary structure follows the pattern of the friction ridges and valleys on the surface. In some other locations on the body, the structure of the capillary bed may be less ordered, but is still characteristic of the particular location and person. As well, the topography of the interface between the different layers of skin is quite complex and characteristic of the skin location and the person. While these sources of subsurface structure of skin and underlying tissue represent a significant noise source for non-imaging optical measurements of skin for biometric determinations or analyte measurements, the structural differences are manifested by spatiospectral features that can be compared favorably through embodiments of the invention.

In some instances, inks, dyes and/or other pigmentation may be present in portions of the skin as topical coating or subsurface tattoos. These forms of artificial pigmentation may or may not be visible to the naked human eye. However, if one or more wavelengths used by the apparatus of the present invention is sensitive to the pigment, the sensor can be used in some embodiments to verify the presence, quantity and/or shape of the pigment in addition to other desired measurement tasks.

In general, embodiments of the present invention provide methods and systems that collect spatiospectral information that may be represented in a multidimensional data structure that has independent spatial and spectral dimensions. In certain instances, the desired information is contained in just a portion of the entire multidimensional data structure. For example, estimation of a uniformly distributed, spectrally active compound may require just the measured spectral characteristics, which may be extracted from the overall multidimensional data structure. In such cases, the overall system design may be simplified to reduce or eliminate the spatial component of the collected data by reducing the number of image pixels, even to a limit of a single pixel. Thus, while the systems and methods disclosed are generally described in the context of spatiospectral imaging, it will be recognized that the invention encompasses similar measurements in which the degree of imaging is greatly reduced, even to the point where there is a single detector element.

## 2. Noncontact Biometric Sensors

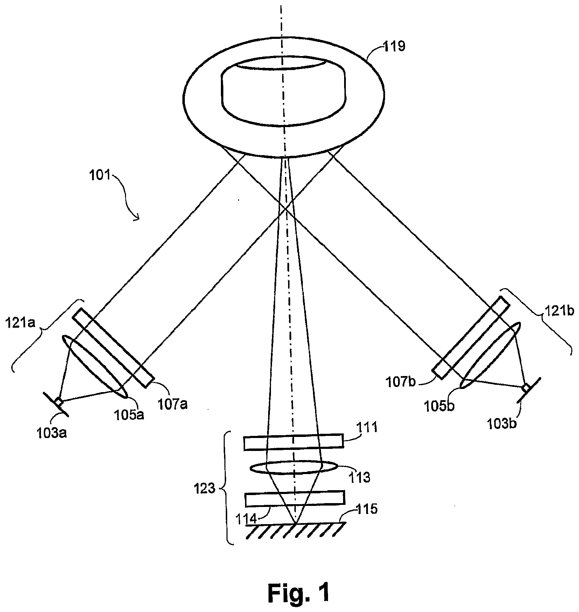

One embodiment of the invention is depicted with the schematic diagram of Fig. 1, which shows a front view of a noncontact biometric sensor 101. The sensor 101 comprises an illumination subsystem 121 having one or more light sources 103 and a detection subsystem 123 with an imager 115. The figure depicts an embodiment in which the illumination subsystem 121 comprises a plurality of illumination subsystems 121a and 121b, but the invention is not limited by the number of illumination or detection subsystems 121 or 123. For example, the number of illumination subsystems 121 may conveniently be selected to achieve certain levels of illumination, to meet packaging requirements, and to meet other structural constraints of the sensor 101. Illumination light passes from the source 103 through illumination optics 105 that shape the illumination to a desired form, such as in the form of flood light, light lines, light points, and the like. The illumination optics 105 are shown for convenience as consisting of a lens but may more generally include any combination of one or more lenses, one or more mirrors, and/or other optical elements. The illumination optics 105 may also comprise a scanner mechanism (not shown) to scan the illumination light in a specified one-dimensional or two-dimensional pattern. The light source 103 may comprise a point source, a line source, an area source, or may comprise a series of such sources in different embodiments. In one embodiment, the illumination light is provided as polarized light, such as by disposing a linear polarizer 107 through which the light passes before striking a finger 119 or other skin site of the person being studied. Embodiments like those shown in Fig. 1 are referred to herein as "noncontact" sensors because the imaged skin site may be positioned to interact with the light without being in contact with any solid surface. In "contact" biometric sensors described in detail below, the imaged skin site is in contact with some solid surface such as a platen or light detector.

In some instances, the light source 103 comprises a white-light source, which maybe provided as a broad-band source or as a collection of narrow-band emitters in different embodiments. Examples of broad-band sources include white-light emitting diodes ("LEDs"), incandescent bulbs or glowbars, and the like. Collections of narrow-band emitters may comprise quasimonochromatic light sources having primary-color wavelengths, such as in an embodiment that includes a red LED or laser diode, a green LED or laser diode, and a blue LED or laser diode.

An alternative mechanism for reducing the directly reflected light makes use of optical polarizers. Both linear and circular polarizers can be employed advantageously to make the optical measurement more sensitive to certain skin depths, as known to on familiar in the art. In the embodiment illustrated in Fig. 1, the illumination light is polarized by linear polarizer 107. The detection subsystem 123 may then also include a linear polarizer 111 that is arranged with its optical axis substantially orthogonal to the illumination polarizer 107. In this way, light from the sample must undergo multiple scattering events to significantly change it state of polarization. Such events occur when the light penetrates the surface of the skin and is scattered back to the detection subsystem 123 after many scatter events.

Conversely, the use of two polarizers 107 and 111 may also be used to increase the influence of directly reflected light by arranging the polarizer 111 to be substantially parallel to polarizer 107. In some systems, it may be advantageous to combine two or more polarization configurations in a single device to enable the collection of multispectral data collected under two different polarization conditions (i.e. under crossed-polarization and under parallel-polarization conditions). In other embodiments, either polarizer 107 or 111, or both, may be omitted, allowing for the collection of substantially randomly polarized light.

The detection subsystem 123 may incorporate detection optics that comprise lenses, mirrors, phase plates and wavefront coding devices, and/or other optical elements that form an image onto the detector 115. The detection optics 113 may also comprise a scanning mechanism (not shown) to relay portions of the overall image onto the detector 115 in sequence. In all cases, the detection subsystem 123 is configured to be sensitive to light that has penetrated the surface of the skin and undergone optical scattering within the skin and/or underlying tissue before exiting the skin.

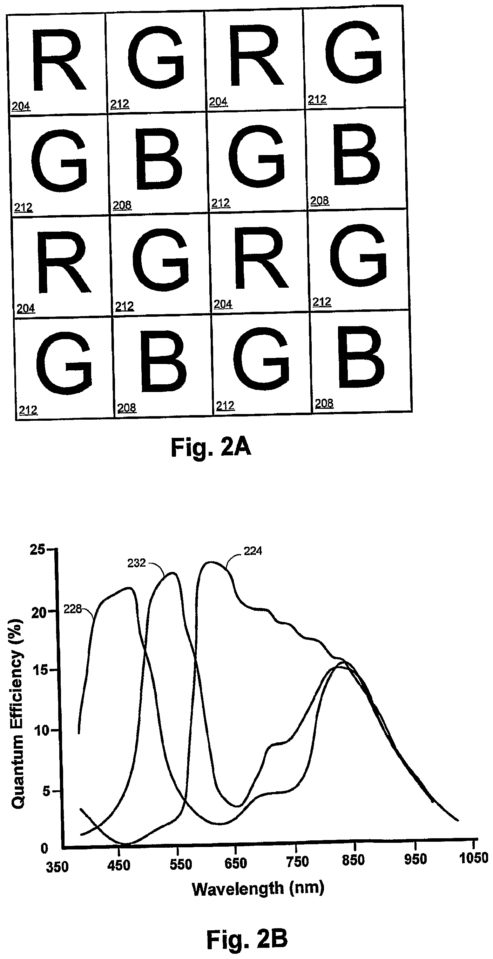

In embodiments where white light is used, the detector 115 may comprise a Bayer color filter array in which filter elements corresponding to a set of primary colors are arranged in a Bayer pattern. An example of such a pattern is shown in Fig. 2A for an arrangement that uses red 204, green 212, and blue 208 color filter elements. In some instances, the detector subsystem 123 may additionally comprise an infrared filter 114 disposed to reduce the amount of infrared light detected. As seen from the color response curve for a typical Bayer filter array shown in Fig. 2B, there is generally some overlap in the spectral ranges of the red 224, green 232, and blue 228 transmission characteristics of the filter elements. As evident particularly in the curves for the green 232 and blue 228 transmission characteristics, the filter array may allow the transmission of infrared light. This is avoided with the inclusion of an infrared filter 114 as part of the detector subsystem. In other embodiments, the infrared filter 114 may be omitted and one or more light sources 103 that emit infrared light may be incorporated. In this way, all color filter elements 204, 208, and 212 may allow the light to substantially pass through, resulting in an infrared image across the entire detector 115.

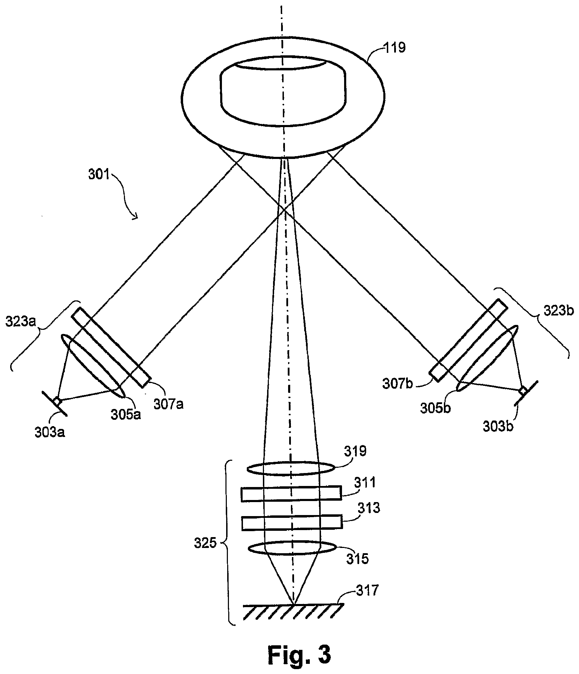

Another embodiment of a noncontact biometric sensor is shown schematically with the front view of Fig. 3. In this embodiment, the biometric sensor 301 comprises an illumination subsystem 323 and a detection subsystem 325. Similar to the embodiment described in connection with Fig. 1, there may be multiple illumination subsystems 323 in some embodiments, with Fig. 3 showing a specific embodiment having two illumination subsystems 323. A white-light source 303 comprised by the illumination subsystem 323 may be any source of white light, including the broad-band or combination of narrow-band sources described above. Light from the white-light source 303 passes through illumination optics 305 and a linear polarizer 307 before passing into the skin site 119. A portion of the light is diffusely reflected from the skin site 119 into the detection subsystem 325, which comprises imaging optics 315 and 319, a linear polarizer 311, and a dispersive optical element 313. The dispersive element 313 may comprise a one- or two-dimensional grating, which may be transmissive or reflective, a prism, or any other optical component known in the art to cause a deviation of the path of light as a function of the light's wavelength. In the illustrated embodiment, the first imaging optics 319 acts to collimate light reflected from the skin site 119 for transmission through the linear polarizer 311 and dispersive element 313. Spectral components of the light are angularly separated by the dispersive element 313 and are separately focused by the second imaging optics 315 onto a detector. As discussed in connection with Fig. 1, when the optical axis of the polarizers 307 and 311 are oriented to be substantially orthogonal to each other, polarizers 307 and 311 respectively comprised by the illumination and detection subsystems 323 and 325 act to reduce the detection of directly reflected light at the detector 317. The polarizers 307, 311 may also be oriented such that their optical axes are substantially parallel, which will increase the detection of directly reflected light at the detector 317. In some embodiments, either polarizer 307 or 311, or both, may be omitted.

The image generated from light received at the detector is thus a "coded" image in the manner of a computer tomographic imaging spectrometer ("CTIS"). Both spectral and spatial information are simultaneously present in the resulting image. The individual spectral patters may be obtained by mathematical inversion or "reconstruction" of the coded image.

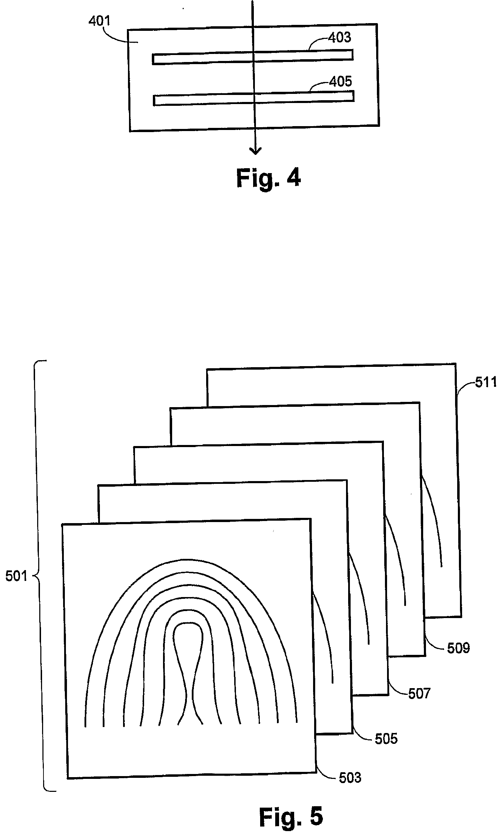

The description of the contactless sensor of Fig. 1 noted that a scanner mechanism may be provided to scan the illumination light. This is an example of a more general class of embodiments in which there is relative motion of the illumination region and skin site. In such embodiments, the image may be constructed by building up separate image portions collected during the relative motion. Such relative motion may also be achieved in embodiments that configure the sensor in a swipe configuration, in which the user is instructed to translate the skin site. One example of a swipe sensor is shown in top view with the schematic illustration of Fig. 4. In this figure, the illumination region and detection region 405 of a sensor 401 are substantially collinear. In some embodiments of a swipe sensor 401, there may be more than a single illumination region. For example, there may be a plurality of illumination regions arranged on either side of the detection region 405. In some embodiments, the illumination region 403 may partially or fully overlay the detection region. The image data are collected with the sensor by translating a finger or other body part through the optically active region, as indicated by the arrow in Fig. 4. A swipe sensor may be implemented with any of the contactless sensor configurations described above, although in some implementations it may be used with a contact configuration, examples of which are described in detail below. The light that is received sequentially from discrete portions of the skin site is used to build up the image that is subsequently used for biometric applications.

The embodiments described above produce a body of spatio-spectral data, which may be used in biometrics applications as described below. The invention is not limited to any particular manner of storing or analyzing the body of spatio-spectral data. For purposes of illustration, it is shown in the form of a datacube in Fig. 5. The datacube 501 is shown decomposed along a spectral dimension with a plurality of planes 503, 505, 507, 509, 511, each of which corresponds to a different portion of the light spectrum and each of which include spatial information. In some instances, the body of spatio-spectral data may include additional types of information beyond spatial and spectral information. For instance, different illumination conditions as defined by different illumination structures, different polarizations, and the like may provide additional dimensions of information. More broadly, data collected under a plurality of optical conditions, whether they be collected simultaneously or sequentially, is referred to herein as "multispectral" data. A more complete description of aspects of multispectral data is described in copending, commonly assigned U.S. Pat. Appl. No. 11/379,945, entitled "MULTISPECTRAL BIOMETRIC SENSORS," filed April 24, 2006. Spatio-spectral data may thus be considered to be a subset of certain types of multispectral data where the different optical conditions include different illumination wavelengths.

In an embodiment where illumination takes place under white light, the images 503, 505, 507, 509, and 511 might correspond, for example to images generated using light at 450 nm, 500 nm, 550 nm, 600 nm, and 650 mn. In another example, there may be three images that correspond to the amount of light in the red, green, and blue spectral bands at each pixel location. Each image represents the optical effects of light of a particular wavelength interacting with skin. Due to the optical properties of skin and skin components that vary by wavelength, each of the multispectral images 503, 505, 507, 509, and 511 will be, in general, different from the others. The datacube may thus be expressed as $R(X_S, Y_S, X_I, Y_I, \lambda)$ and describes the amount of diffusely reflected light of wavelength $\lambda$ seen at each image point $X_I, Y_I$ when illuminated at a source point $X_S, Y_S$. Different illumination configurations (flood, line, etc.) can be summarized by summing the point response over appropriate source point locations. A conventional non-TIR fingerprint image $F(X_I, Y_I)$ can loosely be described as the multispectral data cube for a given wavelength, $\lambda_0$, and summed over all source positions:

$$F(X_I, Y_I) = \sum_{Y_S} \sum_{X_S} R\left(X_S, Y_S, X_I, Y_I, \lambda_0\right).$$

Conversely, the spectral biometric dataset $S(\lambda)$ relates the measured light intensity for a given wavelength $\lambda$ to the difference $\vec{D}$ between the illumination and detection locations:

$$S(\vec{D}, \lambda) = R(X_I - X_S, Y_I - Y_S, \lambda).$$

The datacube R is thus related to both conventional fingerprint images and to spectral biometric datasets. The datacube R is a superset of either of the other two data sets and contains correlations and other information that may be lost in either of the two separate modalities.

The light that passes into the skin and/or underlying tissue is generally affected by different optical properties of the skin and/or underlying tissue at different wavelengths. Two optical effects in the skin and/or underlying tissue that are affected differently at different wavelengths are scatter and absorbance. Optical scatter in skin tissue is generally a smooth and relatively slowly varying function wavelength. Conversely, absorbance in skin is generally a strong function of wavelength due to particular absorbance features of certain components present in the skin. For example blood, melanin, water, carotene, biliruben, ethanol, and glucose all have significant absorbance properties in the spectral region from 400 nm to 2.5 $\mu$m, which may sometimes be encompassed by the white-light sources.

The combined effect of optical absorbance and scatter causes different illumination wavelengths to penetrate the skin to different depths. This effectively causes the different spectral images to have different and complementary information corresponding to different volumes of illuminated tissue. In particular, the capillary layers close to the surface of the skin have distinct spatial characteristics that can be imaged at wavelengths where blood is strongly absorbing. Because of the complex wavelength-dependent properties of skin and underlying tissue, the set of spectral values corresponding to a given image location has spectral characteristics that are well-defined and distinct. These spectral characteristics may be used to classify the collected image on a pixel-by-pixel basis. This assessment may be performed by generating typical tissue spectral qualities from a set of qualified images. For example, the spatio-spectral data shown in Fig. 5 may be reordered as an $N \times 5$ matrix, where $N$ is the number of image pixels that contain data from living tissue, rather than from a surrounding region of air. An eigenanalysis or other factor analysis performed on this set matrix produces the representative spectral features of these tissue pixels. The spectra of pixels in a later data set may then be compared to such previously established spectral features using metrics such as Mahalanobis distance and spectral residuals. If more than a small number of image pixels have spectral qualities that are inconsistent with living tissue, then the sample is deemed to be non-genuine and rejected, thus providing a mechanism for incorporating antispoofing methods in the sensor based on determinations of the liveness of the sample.

Alternatively, textural characteristics of the skin may alone or in conjunction with the spectral characteristics be used to determine the authenticity of the sample. For example, each spectral image may be analyzed in such a way that the magnitude of various spatial characteristics may be described. Methods for doing so include wavelet transforms, Fourier transforms, cosine transforms, gray-level co-occurrence, and the like. The resulting coefficients from any such transform described an aspect of the texture of the image from which they were derived. The set of such coefficients derived from a set of spectral images thus results in a description of the chromatic textural characteristics of the multispectral data. These characteristics may then be compared to similar characteristics of known samples to perform a biometric determination such as spoof or liveness determination. Methods for performing such determinations are generally similar to the methods described for the spectral characteristics above. Applicable classification techniques for such determinations include linear and quadratic discriminant analysis, classification trees, neural networks, and other methods known to those familiar in the art.

Similarly, in an embodiment where the sample is a volar surface of a hand or finger, the image pixels may be classified as "ridge," "valley," or "other" based on their spectral qualities or their chromatic textural qualities. This classification can be performed using discriminant analysis methods such as linear discriminant analysis, quadratic discriminant analysis, principal component analysis, neural networks, and others known to those of skill in the art. Since ridge and valley pixels are contiguous on a typical volar surface, in some instances, data from the local neighborhood around the image pixel of interest are used to classify the image pixel. In this way, a conventional fingerprint image may be extracted for further processing and biometric assessment. The "other" category may indicate image pixels that have spectral qualities that are different than anticipated in a genuine sample. A threshold on the total number of pixels in an image classified as "other" may be set. If this threshold is exceeded, the sample may be determined to be non-genuine and appropriate indications made and actions taken.

In a similar way, multispectral data collected from regions such as the volar surface of fingers may be analyzed to directly estimate the locations of "minutiae points," which are defined as the locations at which ridges end, bifurcate, or undergo other such topographic change. For example, the chromatic textural qualities of the multispectral dataset may be determined in the manner described above. These qualities may then be used to classify each image location as "ridge ending," "ridge bifurcation," or "other" in the manner described previously. In this way, minutiae feature extraction may be accomplished directly from the multispectral data without having to perform computationally laborious calculations such as image normalization, image binarization, image thinning, and minutiae filtering, techniques that are known to those familiar in the art.

Biometric determinations of identity may be made using the entire body of spatio-spectral data or using particular portions thereof. For example, appropriate spatial filters may be applied to separate out the lower spatial frequency information that is typically representative of deeper spectrally active structures in the tissue. The fingerprint data may be extracted using similar spatial frequency separation and/or the pixel-classification methods disclosed above. The spectral information can be separated from the active portion of the image in the manner discussed above. These three portions of the body of spatio-spectral data may then be processed and compared to the corresponding enrollment data using methods known to one familiar in the art to determine the degree of match. Based upon the strength of match of these characteristics, a decision can be made regarding the match of the sample with the enrolled data. Additional details regarding certain types of spatio-spectral analyses that may be performed are provided in U.S. Pat. Appl. No. 10/818,698, entitled "MULTISPECTRAL BIOMETRIC SENSOR," filed April 5, 2004 by Robert K. Rowe et al.,

As previously noted, certain substances that may be present in the skin and underlying tissue have distinct absorbance characteristics. For example, ethanol has characteristic absorbance peaks at approximately 2.26 $\mu$m, 2.30 $\mu$m, and 2.35 $\mu$m, and spectral troughs at 2.23 $\mu$m, 2.28 $\mu$m, 2.32 $\mu$m, and 2.38 $\mu$m. In some embodiments, noninvasive optical measurements are performed at wavelengths in the range of 2.1 - 2.5 $\mu$m, more particularly in the range of 2.2 - 2.4 $\mu$m. In an embodiment that includes at least one of the peak wavelengths and one of the trough wavelengths, the resulting spectral data are analyzed using multivariate techniques such as partial least squares, principal-component regression, and others known to those of skill in the art, to provide an estimate of the concentration of alcohol in the tissue, as well as to provide a biometric signature of the person being tested. While a correlation to blood-alcohol level may be made with values determined for a subset of these wavelengths, it is preferable to test at least the three spectral peak values, with more accurate results being obtained when the seven spectral peak and trough values are measured.

In other embodiments, noninvasive optical measurements are performed at wavelengths in the range of 1.5 - 1.9 $\mu$m, more particularly in the range of 1.6 - 1.8 $\mu$m. In specific embodiments, optical measurements are performed at one or more wavelengths of approximately 1.67 $\mu$m, 1.69 $\mu$m, 1.71 $\mu$m, 1.73 $\mu$m, 1.74 $\mu$m 1.76 $\mu$m and 1.78 $\mu$m. The presence of alcohol is characterized at these wavelengths by spectral peaks at 1.69 $\mu$m, 1.73 $\mu$m, and 1.76 $\mu$m and by spectral troughs at 1.67 $\mu$m, 1.71 $\mu$m, 1.74 $\mu$m, and 1.78 $\mu$m. Similar to the 2.1 - 2.5 $\mu$m wavelength range, the concentration of alcohol is characterized by relative strengths of one or more of the spectral peak and trough values. Also, while a correlation to blood-alcohol level may be made with values determined for a subset of these wavelengths in the 1.5 - 1.9 $\mu$m range, it is preferable to test at least the three spectral peak values, with more accurate results being obtained when the seven spectral peak and trough values are measured.

A small spectral alcohol-monitoring device may be embedded in a variety of systems and applications in certain embodiments. The spectral alcohol-monitoring device can be configured as a dedicated system such as may be provided to law-enforcement personnel, or may be integrated as part of an electronic device such as an electronic fob, wristwatch, cellular telephone, PDA, or any other electronic device, for an individual's personal use. Such devices may include mechanisms for indicating to an individual whether his blood-alcohol level is within defined limits. For instance, the device may include red and green LEDs, with electronics in the device illuminating the green LED if the individual's blood-alcohol level is within defined limits and illuminating the red LED if it is not. In one embodiment, the alcohol-monitoring device may be included in a motor vehicle, typically positioned so that an individual may conveniently place tissue, such as a fingertip, on the device. While in some instances, the device may function only as an informational guide indicating acceptability to drive, in other instances ignition of the motor vehicle may affirmatively depend on there being a determination that the individual has a blood-alcohol level less than a prescribed level.

3. Contact Biometric Sensors

Biometric sensors may be constructed in a fashion similar to that shown in Figs. 1 and 3, but configured so that the skin site is placed in contact with a platen. Such designs have certain additional characteristics that result from the interaction of light with the platen, sometimes permitting additional information to be incorporated as part of the collect spatio-spectral data.

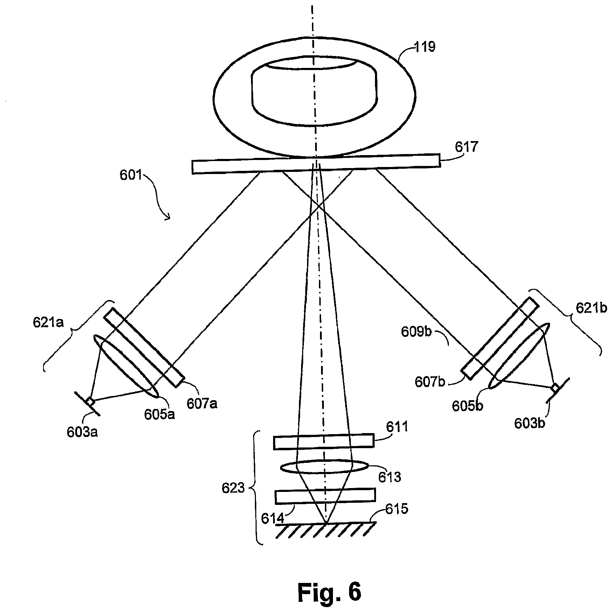

One embodiment is shown in Fig. 6, which provides a front view of a contact biometric sensor 601. Like the sensor illustrated in Fig. 1, the contact sensor 601 has one or more illumination subsystems 621 and a detection subsystem 623. Each of the illumination subsystems 621 comprises one or more white-light sources 603 and illumination optics that shape the light provided by the sources 603 into a desired form. As with the non-contact arrangements, the illumination optics may generally include any combination of optical elements and may sometimes include a scanner mechanism. In some instances, the illumination light is provided as polarized light by disposing a polarizer 607 through which the illumination light passes. Examples of white-light sources 603, including broad- and narrow-band sources were described above, and the sources 603 may be configured to provide sources having different shapes in different embodiments.

The illumination light is directed by the illumination optics 621 to pass through a platen 617 and illuminate the skin site 119. The sensor layout 601 and components may advantageously be selected to minimize the direct reflection of the illumination optics 621. In one embodiment, such direct reflections are reduced by relatively orienting the illumination subsystem 621 and detection subsystem 623 such that the amount of directly reflected light detected is minimized. For instance, optical axes of the illumination subsystem 621 and the detection subsystem 623 may be placed at angles such that a mirror placed on the platen 617 does not direct an appreciable amount of illumination light into the detection subsystem 623. In addition, the optical axes of the illumination and detection subsystems 621 and 623 may be placed at angles relative to the platen 617 such that the angular acceptance of both subsystems is less than the critical angle of the system 601; such a configuration avoids appreciable effects due to total internal reflectance between the platen 617 and the skin site 119.

The presence of the platen 617 does not adversely interfere with the ability to reduce the directly reflected light by use of polarizers. The detection subsystem 623 may include a polarizer 611 having an optical axis substantially orthogonal or parallel to the polarizer 607 comprised by the illumination subsystem 621. Surface reflections at the interface between the platen 617 and the skin site 119 are reduced in the case where polarizers 611 and 607 are oriented substantially orthogonal to each other since light from the sample must undergo sufficiently many scattering events to change its state of polarization before it can be sensed by the detector 615. The detection subsystem 623 may additionally incorporate detection optics that form an image of the region near the platen surface 617 onto the detector 615. In one embodiment, the detection optics 613 comprise a scanning mechanism (not shown) to relay portions of the platen region onto the detector 615 in sequence. An infrared filter 614 may be included to reduce the amount of infrared light detected, particularly in embodiments where the detector 615 is sensitive to infrared light, such as when a Bayer filter array is used. Conversely, as described above, the infrared filter 614 may be omitted in some embodiments and an additional light source 603 with emissions in the infrared may be included in some embodiments.

As in the other arrangements described above, the detection subsystem 623 is generally configured to be sensitive to light that has penetrated the surface of the skin and undergone optical scattering within the skin and/or underlying tissue. The polarizers may sometimes be used to create or accentuate the surface features. For instance, if the illumination light is polarized in a direction parallel ("P") with the platen 617, and the detection subsystem 623 incorporates a polarizer 611 in a perpendicular orientation ("S"), then the reflected light is blocked by as much as the extinction ratio of the polarizer pair. However, light that crosses into the skin site at a ridge point is optically scattered, which effectively randomizes the polarization (though the skin does have some characteristic polarization qualities of its own, as is known to those of skill in the art). This allows a portion, on the order of 50%, of the absorbed and re-emitted light to be observed by the S-polarized imaging system.

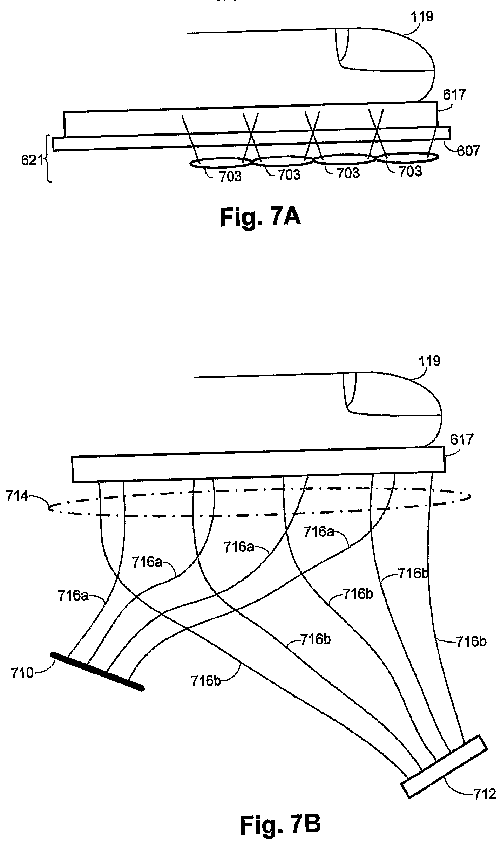

A side view of one of the embodiments of the invention is shown with the schematic drawing provided in Fig. 7A. For clarity, this view does not show the detection subsystem, but does show an illumination subsystem 621 explicitly. The illumination subsystem 621 in this embodiment has a plurality of white-light sources 703 that are distributed spatially. As shown in the drawing, the illumination optics 621 are configured to provide flood illumination, but in alternative embodiments could be arranged to provide line, point, or other patterned illumination by incorporation of cylindrical optics, focusing optics, or other optical components as known to those knowledgeable in the art.

The array of white-light sources 703 in Fig. 7A need not actually be planar as shown in the drawing. For example, in other embodiments, optical fibers, fiber bundles, or fiber optical faceplates or tapers could convey the light from the light sources at some convenient locations to an illumination plane, where light is reimaged onto the skin site 119. The light sources could be controlled turning the drive currents on and off as LEDs might be. Alternatively, if an incandescent source is used, switching of the light may be accomplished using some form of spatial light modulator such as a liquid crystal modulator or using microelectromechanical-systems ("MEMS") technology to control apertures, mirrors, or other such optical elements. Such configurations may allow the structure of the sensor to be simplified. One embodiment is illustrated in Fig. 7B, which shows the use of optical fibers and electronic scanning of illumination sources such as LEDs. Individual fibers 716a connect each of the LEDs located at an illumination array 710 to an imaging surface, and other fibers 716b relay the reflected light back to the imaging device 712, which may comprise a photodiode array, CMOS array, or CCD array. The set of fibers 716a and 716b thus defines an optical fiber bundle 714 used in relaying light.

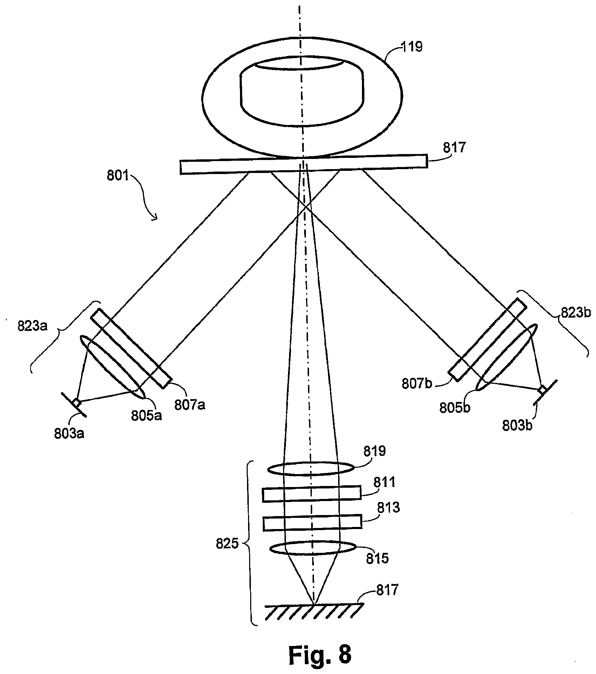

Another embodiment of a contact biometric sensor is shown schematically with the front view of Fig. 8. In this embodiment, the biometric sensor 801 comprises one or more white-light illumination subsystems 823 and a detection subsystem 825. The illumination subsystems 823 comprise a white-light source 803 that provides light that passes through illumination optics 805 and a polarizer 807 to be directed to a platen 817 over which a skin site is disposed 119. A portion of the light is diffusely reflected from the skin site 119 into the detection subsystem 825, which comprises imaging optics 815 and 819, a crossed polarizer 811, and a dispersive optical element 813. The first imaging optics 819 collimate light reflected from the skin site 119 for transmission through the crossed polarizer 811 and dispersive element 813. Separated spectral components are separately focused onto the detector 817 by the second imaging optics 815.

Contact biometric sensors like those illustrated in Figs. 6 - 8 are also amenable to configurations in which the illumination region is in relative motion with the skin site. As previously noted, such relative motion may be implemented with a mechanism for scanning the illumination light and/or by moving the skin site. The presence of a platen in contact-sensor embodiments generally facilitates motion of the skin site by confining a surface of the skin site to a defined plane; in embodiments where freedom of motion is permitted in three dimensions, additional difficulties may result from movement of the skin site outside the imaging depth. A swipe sensor may accordingly be implemented with contact biometric sensors in a fashion as generally described in connection with Fig. 4 above, but with a platen that prevents motion of the skin site in one direction. While in some embodiments, the swipe sensor may be a stationary system, a contact configuration allows a roller system to be implemented in which the skin site is rolled over a roller structure that is transparent to the white light. An encoder may record position information and aid in stitching a full two-dimensional image from a resulting series of image slices, as understood by those of skill in the art. Light received from discrete portions of the skin site is used to build up the image.

While the above descriptions of noncontact and contact biometric sensors have focused on embodiments in which white light is used, other embodiments may make use of other spectral combinations of light in similar structural arrangements. In addition, other embodiments may include additional variations in optical conditions to provide multispectral conditions. Some description of such multispectral applications is provided in commonly assigned U.S. Pat. Appl. No. 10/818,698, entitled "MULTISPECTRAL BIOMETRIC SENSOR," filed April 5, 2004 by Robert K. Rowe et al.; U.S. Pat. 11/177,817, entitled "LIVENESS SENSOR," filed July 8, 2005 by Robert K. Rowe; U.S. Prov. Pat. No. 60/576,364, entitled "MULTISPECTRAL FINGER RECOGNITION," filed June 1, 2004 by Robert K. Rowe and Stephen P. Corcoran; U.S. Prov. Pat. Appl. No. 60/600,867, entitled "MULTISPECTRAL IMAGING BIOMETRIC," filed August 11, 2004 by Robert K. Rowe; U.S. Pat. No. 11/115,100, entitled "MULTISPECTRAL IMAGING BIOMETRICS," filed April 25, 2005 by Robert K. Rowe; U.S. Pat. Appl. No. 11/115,101, entitled "MULTISPECTRAL BIOMETRIC IMAGING," filed April 25, 2005; U.S. Pat. No. 11/115,075, entitled "MULTISPECTRAL LIVENESS DETERMINATION," filed April 25, 2005; U.S. Prov. Pat. Appl. No. 60/659,024, entitled "MULTISPECTRAL IMAGING OF THE FINGER FOR BIOMETRICS," filed March 4, 2005 by Robert K. Rowe et al.; U.S. Prov. Pat. Appl. No. 60/675,776, entitled "MULTISPECTRAL BIOMETRIC SENSORS," filed April 27, 2005 by Robert K. Rowe; and U.S. Pat. Appl. No. 11/379,945, entitled "MULTISPECTRAL BIOMETRIC SENSORS," filed April 24, 2006 by Robert K. Rowe.

The noncontact and contact biometric sensors described above use white-light imaging in certain embodiments. The use of white light permits images to be collected simultaneously at multiple colors, with the overall speed of data collection being faster than in embodiments where discrete states are collected separately. This reduced data-collection time leads to a reduction in motion artifacts as the skin site moves during data collection. The overall sensor size may also be reduced and provided at lower cost by using a smaller number of light sources when compared with the use of discrete illumination sources for different colors. Corresponding reductions are also possible in the electronics used to support coordinated operation of the light sources. In addition, color imagers are currently available at prices that are typically lower than monochrome imagers.

The use of white-light imaging also permits a reduction in data volume when the sensor is designed to use all pixels in achieving the desired resolution. For instance, a typical design criterion may provide a 1-inch field with a 500 dots-per-inch resolution. This can be achieved with a monochrome camera having 500 $\times$500 pixels. It can also be achieved with a 1000 $\times$ 1000 color camera when extracting each color plane separately. The same resolution can be achieved by using a 500 × 500 color imager and converting to {R, G, B} triplets and then extracting the monochrome portion of the image. This is a specific example of a more general procedure in which a color imager is used by converting to primary-color triplets, followed by extraction of a monochrome portion of an image. Such a procedure generally permits a desired resolution to be achieved more efficiently than with other extraction techniques.

4. Integrated Multispectral / TIR Sensor

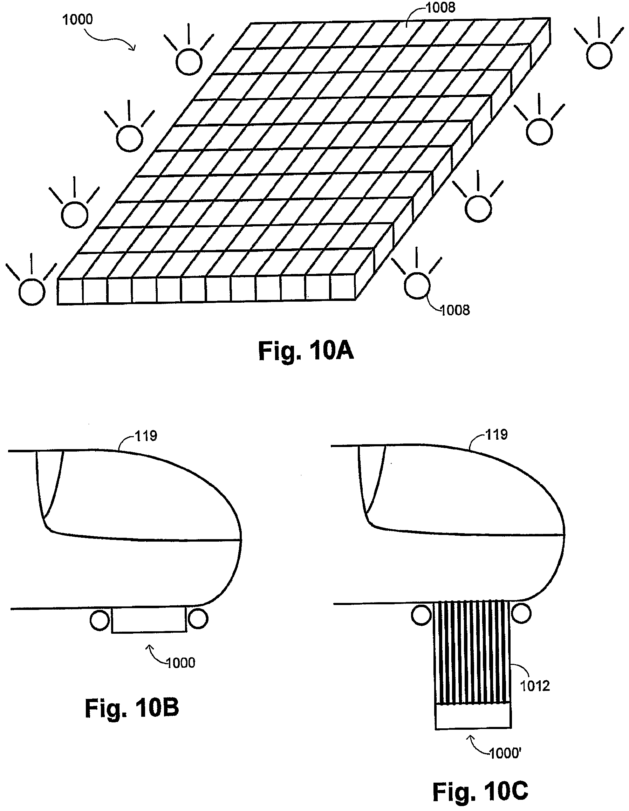

In some embodiments, the multispectral imaging enabled by the sensor described above may be integrated with a conventional TIR imaging system. One illustration of such an integration is provided in Fig. 10, which shows a bright-field fingerprint sensor integrated with multispectral-analysis capability. The sensor comprises a light source 953, a platen 961, and an imaging system 955. The imaging system 965 may comprise lenses, mirrors, optical filters, a digital imaging array, and other such optical elements (not shown). The optical axis of the imaging system 955 is at an angle greater than the critical angle with respect to surface 961a. The light from the source 953 passes into the platen 961 and strikes a diffuse reflective coating 963, which broadly illuminates the platen surface 961a. In the absence of a finger 969, light undergoes TIR at surface 961a and a portion is collected and forms a uniformly bright TIR image by imaging system 955. When skin of proper index of refraction is in optical contact with the platen surface 961a, the points of contact will form relatively dark regions on the resulting image. Variants of such a configuration, using different locations for the sources 953 and/or imaging systems 955, may achieve a dark-field TIR image. For example, if the platen 961 is illuminated with light source 957 and imaged by imaging system 955, a dark-field image is produced, wherein points of contact are relatively bright. In some dark-field embodiments, coating 963 may comprise an optical absorber.
A second imaging system 959 looks up at the finger 969 through facet 961b. Imaging system 959 has an optical axis less than the critical angle with respect to facet 961a. In some embodiments, imaging system 959 is oriented approximately normal to facet 961a. This imaging system may comprise lenses, mirrors, optical filters, and a digital imaging array (not shown). In this manner, when light source 953 is illuminated, a TIR image may be captured by camera 955 while a direct image may be captured by camera 959. Even in cases where the TIR image is adversely affected by water, dirt, lack of contact, dry skin, etc., the image captured by camera 959 is relatively unaffected and generally contains usable biometric features, including the fingerprint pattern.
Imaging system 959 may further incorporate an optical polarizer (not shown), which may be a linear polarizer or elliptical (e.g. circular) polarizer. As well, other light sources 957 may be added to the system. The light sources 957 may be incandescent sources such as quartz-tungsten-halogen lamps or others commonly known in the art. The sources 957 may be other broadband sources such as white-light LEDs or others known in the art. The sources may be quasimonochromatic sources such as solid-state LEDs, organic LEDs, laser diodes, or other kinds of lasers or quasimonochromatic sources known in the art. The sources 957 may further comprise lenses, mirrors, optical diffusers, optical filters, and other such optical elements.
The sources 957 may be substantially the same or may provide for different illumination wavelengths, angles, and/or polarization conditions. In the latter case, one of the sources 957a may have an optical polarizer (not shown) that is oriented substantially orthogonal to the polarizer incorporated in the imaging system 959. Such an optical geometry tends to emphasize features of the skin that lie below the surface. One of the light sources 957b may incorporate a polarizer that is substantially parallel to the polarizer used in imaging system 959, which will tend to emphasize surface features of the skin. Alternatively, one of the light sources 957b may omit the polarizer, producing random polarization. Such random polarization may be described as a combination of polarization parallel and perpendicular to the polarizer in the imaging system 959, producing an image that combines features from the two polarization conditions. The light sources 957 may be of the same wavelength or of different wavelengths (with or without polarizers). The number and arrangement of sources 957 may be different for different embodiments to accommodate form-factor constraints, illumination-level constraints, and other product requirements.
In one embodiment, the sources 957 are oriented at an angle less than the critical angle with respect to facet 961a. In a preferred embodiment, sources may be located at such an angle and position that no direct reflection of the source is seen by imaging system 959 or 955. Such direct reflections can also be greatly mitigated through the use of crossed-polarizer configurations, but some image artifacts will still be generally present if the sources are in the field of view. Moreover, parallel-polarized and nonpolarized configurations are very susceptible to such back reflections.
Attempts to spoof such a system may generally be characterized according to three different classes: (1) use of a thin transparent film overlying the finger or other tissue; (2) use of a thin scattering film overlying the finger or other tissue; and (3) use of a thick sample overlying the finger or other tissue. Embodiments of the invention are of greater utility in the second and third instances, i.e. when the spoof comprises a thick film or a thin scattering film. In instances where the spoof comprises a thin transparent film, i.e. is substantially nonabsorbing and nonscattering, an attempt to incorporate a topology of a different fingerprint pattern may be detected by comparing conventional and TIR patterns; a more detailed description of how to perform such comparisons is provided in U.S. Pat. Appl. No. 11/015,732.
A spoof that uses a thin scattering film, i.e. a substantially nonabsorbing film with a significant amount of optical scattering, may also incorporate a topology of a different fingerprint pattern in an attempt to fool a conventional sensor. The spectral imaging described herein permits properties of the underlying tissue to be imaged even while the image of the real fingerprint is obscured. Conversely, a thick sample provides a combination of sample scattering and absorbance properties that ensure that detected light does not penetrate significantly into the adjacent tissue, if at all. An attempt to incorporate a topology of a different fingerprint pattern to fool a conventional sensor may be detected in accordance with embodiments of the invention by evaluating the spectral and other optical properties of the thick sample and comparing them to those of real tissue.

5. Texture Biometric Sensor

Another form of contact biometric sensor provided in embodiments of the invention is a texture biometric sensor. "Image texture" refers generally to any of a large number of metrics that describe some aspect of a spatial distribution of tonal characteristics of an image, some of which were described above. For example, some textures, such as those commonly found in fingerprint patterns or wood grain, are flowlike and may be well described by metrics such as an orientation and coherence. For textures that have a spatial regularity (at least locally), certain characteristics of the Fourier transform and the associated power spectrum are important such as energy compactness, dominant frequencies and orientations, etc. Certain statistical moments such as mean, variance, skew, and kurtosis may be used to describe texture. Moment invariants may be used, which are combinations of various moments that are invariant to changes in scale, rotation, and other perturbations. Gray-tone spatial dependence matrices may be generated and analyzed to describe image texture. The entropy over an image region may be calculated as a measure of image texture. Various types of wavelet transforms may be used to describe aspects of the image texture. Steerable pyramids, Gabor filters, and other mechanisms of using spatially bounded basis functions may be used to describe the image texture. These and other such measures of texture known to one familiar in the art may be used individually or in combination in embodiments of the invention.

Image texture may thus be manifested by variations in pixel intensities across an image, which may be used in embodiments of the invention to perform biometric functions. In some embodiments, additional information may be extracted when such textural analysis is performed for different spectral images extracted from a multispectral data set, producing a chromatic textural description of the skin site. These embodiments advantageously enable biometric functions to be performed by capturing a portion of an image of a skin site. The texture characteristics of the skin site are expected to be approximately consistent over the skin site, permitting biometric functions to be performed with measurements made at different portions of the image site. In many instances, it is not even required that the portions of the skin site used in different measurements overlap with each other.

This ability to use different portions of the skin site provides considerable flexibility in the structural designs that may be used. This is, in part, a consequence of the fact that biometric matching may be performed statistically instead of requiring a match to a deterministic spatial pattern. The sensor may be configured in a compact manner because it need not acquire an image over a specified spatial area. The ability to provide a small sensor also permits the sensor to be made more economically than sensors that need to collect complete spatial information to perform a biometric function. In different embodiments, biometric functions may be performed with purely spectral information, while in other embodiments, spatio-spectral information is used.

One example of a structure for a texture biometric sensor is shown schematically in Fig. 10A. The sensor 1000 comprises a plurality of light sources 1004 and an imager 1008. In some embodiments, the light sources 1004 comprise white-light sources, although in other embodiments, the light sources comprise quasimonochromatic sources. Similarly, the imager 1008 may comprise a monochromatic or color imager, one example of which is an imager having a Bayer pattern. The sensor 1000 is referred to herein as a "contact" sensor because the image is collected substantially in the plane of the skin site 119 being measured. It is possible, however, to have different configurations for operating the sensor, some with the imager 1008 substantially in contact with the skin site 119 and some with the imager 1008 displaced from the plane of the skin site 119.

This is shown for two illustrative embodiments in Figs. 10B and 10C. In the embodiment of Fig. 10B, the imager 1008 is substantially in contact with the skin site 119. Light from the sources 1004 propagates beneath the tissue of the skin site 119, permitting light scattered from the skin site 119 and in the underlying tissue to be detected by the imager 1008. An alternative embodiment in which the imager 1008 is displaced from the skin site 119 is shown schematically in Fig. 10C. In this drawing the sensor 1000' includes an optical arrangement 1012 that translates an image at the plane of the skin site 119 to the imager could comprise a plurality of optical fibers, which translate individual pixels of an image by total internal reflection along the fiber without substantially loss of intensity. In this way, the light pattern detected by the imager 1008 is substantially the same as the light pattern formed at the plane of the skin site 119. The sensor 1000' may thus operate in substantially the same fashion as the sensor 1000 shown in Fig. 10B. That is, light from the sources 1004 is propagated to the skin site, where it is reflected and scattered by underlying tissue after penetrating the skin site 119. Because information is merely translated substantially without loss, the image formed by the imager 1008 in such an embodiment is substantially identical to the image that would be formed with an arrangement like that in Fig. 10A.

In embodiments where purely spectral information is used to perform a biometric function, spectral characteristics in the received data are identified and compared with an enrollment database of spectra. The resultant tissue spectrum of a particular individual includes unique spectral features and combinations of spectral features that can be used to identify individuals once a device has been trained to extract the relevant spectral features. Extraction of relevant spectral features may be performed with a number of different techniques, including discriminant analysis techniques. While not readily apparent in visual analysis of a spectral output, such analytical techniques can repeatably extract unique features that can be discriminated to perform a biometric function. Examples of specific techniques are disclosed in commonly assigned U.S. Pat. No. 6,560,352, entitled "APPARATUS AND METHOD OF BIOMETRIC IDENTIFICATION AND VERIFICATION OF INDIVIDUALS USING OPTICAL SPECTROSCOPY"; U.S. Pat. No. 6,816,605, entitled "METHODS AND SYSTEMS FOR BIOMETRIC IDENTIFICATION OF INDIVIDUALS USING LINEAR OPTICAL SPECTROSCOPY"; U.S. Pat. No. 6,628,809, entitled "APPARATUS AND METHOD FOR IDENTIFICATION OF INDIVIDUALS BY NEAR-INFRARED SPECTROSCOPY"; U.S. Pat. Appl. No. 10/660,884, entitled "APPARATUS AND METHOD FOR IDENTIFICATION OF INDIVIDUAL BY NEAR-INFRARED SPECTROSCOPY," filed September 12, 2003 by Robert K. Rowe et al.; and U.S. Pat. Appl. No. 09/874,740, entitled "APPARATUS AND METHOD OF BIOMETRIC DETERMINATION USING SPECIALIZED OPTICAL SPECTROSCOPY SYSTEM," filed June 5, 2001 by Robert K. Rowe et al.

The ability to perform biometric functions with image-texture information, including biometric identifications, may exploit the fact that a significant portion of the signal from a living body is due to capillary blood. For example, when the skin site 119 comprises a finger, a known physiological characteristic is that the capillaries in the finger follow the pattern of the external fingerprint ridge structure. Therefore, the contrast of the fingerprint features relative to the illumination wavelength is related to the spectral features of blood. In particular, the contrast of images taken with wavelengths longer than about 580 nm are significantly reduced relative to those images taken with wavelengths less than about 580 nm. Fingerprint patterns generated with nonblood pigments and other optical effects such as Fresnel reflectance have a different spectral contrast.

Light scattered from a skin site 119 may be subjected to variety of different types of comparative texture analyses in different embodiments. Some embodiments make use of a form of moving-window analysis of image data derived from the collected light to generate a figure of merit, and thereby evaluate the measure of texture or figure of merit. In some embodiments, the moving window operation may be replaced with a block-by-block or tiled analysis. In some embodiments, a single region of the image or the whole image may be analyzed at one time.

In one embodiment, fast-Fourier transforms are performed on one or more regions of the image data. An in-band contrast figure of merit C is generated in such embodiments as the ratio of the average or DC power to in-band power. Specifically, for an index i that corresponds to one of a plurality of wavelengths comprised by the white light, the contrast figure of merit is

$$C_i \equiv \frac{\sum_\xi \sum_\eta |F_i(\xi,\eta)|^2 \big|_{R_{\text{low}}^2 < (\xi^2+\eta^2) < R_{\text{high}}^2}}{|F_i(0,0)|^2}.$$

In this expression, $F_i(\xi,\eta)$ is the Fourier transform of the image $f_i(x,y)$ at the wavelength corresponding to index $i$, where $x$ and $y$ are spatial coordinates for the image. The range defined by $R_{\text{low}}$ and $R_{\text{high}}$ represents a limit on spatial frequencies of interest for fingerprint features. For example, $R_{\text{low}}$ may be approximately 1.5 fringes/mm in one embodiment and $R_{\text{high}}$ may be 3.0 fringes/mm. In an alternative formulation, the contrast figure of merit may be defined as the ratio of the integrated power in two different spatial frequency bands. The equation shown above is a specific case where one of the bands comprises only the DC spatial frequency.

In another embodiment, moving-window means and moving-window standard deviations are calculated for the collected body of data and used to generate the figure of merit. In this embodiment, for each wavelength corresponding to index i, the moving-window mean $\mu_I$ and the moving-window standard deviation $\sigma_I$ are calculated from the collected image $f_i(x, y)$. The moving windows for each calculation may be the same size and may conveniently be chosen to span on the order of 2 - 3 fingerprint ridges. Preferably, the window size is sufficiently large to remove the fingerprint features but sufficiently small to have background variations persist. The figure of merit $C_i$ in this embodiment is calculated as the ratio of the moving-window standard deviation to the moving-window mean:

$$C_i = \frac{\sigma_i}{\mu_i}.$$

In still another embodiment, a similar process is performed but a moving-window range (*i.e.,* max(image values) - min(image values)) is used instead of a moving-window standard deviation. Thus, similar to the previous embodiment, a moving-window mean $\mu_I$ and a moving-window range $\delta_I$ are calculated from the collected image $f_i(x,y)$ for each wavelength corresponding to index i. The window size for calculation of the moving-window mean is again preferably large enough to remove the fingerprint features but small enough to maintain background variations. In some instances, the window size for calculation of the moving-window mean is the same as for calculation of the moving-window range, a suitable value in one embodiment spanning on the order of 2 - 3 fingerprint ridges. The figure of merit in this embodiment is calculated as the ratio of the moving-window mean:

$$C_i = \frac{\delta_i}{\mu_i}.$$

This embodiment and the preceding one may be considered to be specific cases of a more general embodiment in which moving-window calculations are performed on the collected data to calculate a moving-window centrality measure and a moving-window variability measure. The specific embodiments illustrate cases in which the centrality measure comprises an unweighted mean, but may more generally comprise any other type of statistical centrality measure such as a weighted mean or median in certain embodiments. Similarly, the specific embodiments illustrate cases in which the variability measure comprises a standard deviation or a range, but may more generally comprise any other type of statistical variability measure such as a median absolute deviation or standard error of the mean in certain embodiments.

In another embodiment that does not use explicit moving-window analysis, a wavelet analysis may be performed on each of the spectral images. In some embodiments, the wavelet analysis may be performed in a way that the resulting coefficients are approximately spatially invariant. This may be accomplished by performing an undecimated wavelet decomposition, applying a dual-tree complex wavelet method, or other methods of the sort. Gabor filters, steerable pyramids and other decompositions of the sort may also be applied to produce similar coefficients. Whatever method of decomposition is chosen, the result is a collection of coefficients that are proportional to the magnitude of the variation corresponding to a particular basis function at a particular position on the image. To perform spoof detection, the wavelet coefficients, or some derived summary thereof, may be compared to the coefficients expected for genuine samples. If the comparison shows that the results are sufficiently close, the sample is deemed authentic. Otherwise, the sample is determined to be a spoof. In a similar manner, the coefficients may also be used for biometric verification by comparing the currently measured set of coefficients to a previously recorded set from the reputedly same person.

6. Exemplary Applications

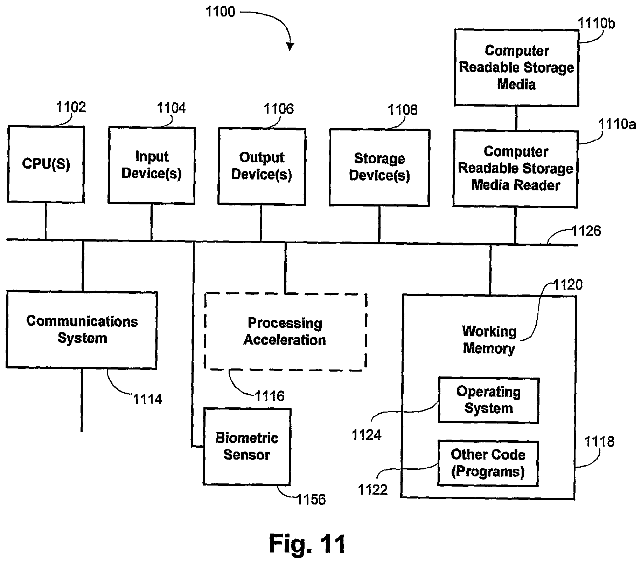

In various embodiments, a biometric sensor, whether it be a noncontact, contact, or texture sensor of any of the types described above, may be operated by a computational system to implement biometric functionality. Fig. 11 broadly illustrates how individual system elements may be implemented in a separated or more integrated manner. The computational device 1100 is shown comprised of hardware elements that are electrically coupled via bus 1126, which is also coupled with the biometric sensor 1156. The hardware elements include a processor 1102, an input device 1104, an output device 1106, a storage device 1108, a computer-readable storage media reader 1110a, a communications system 1114, a processing acceleration unit 1116 such as a DSP or special-purpose processor, and a memory 1118. The computer-readable storage media reader 1110a is further connected to a computer-readable storage medium 1110b, the combination comprehensively representing remote, local, fixed, and/or removable storage devices plus storage media for temporarily and/or more permanently containing computer-readable information. The communications system 1114 may comprise a wired, wireless, modem, and/or other type of interfacing connection and permits data to be exchanged with external devices.

The computational device 1100 also comprises software elements, shown as being currently located within working memory 1120, including an operating system 1124 and other code 1122, such as a program designed to implement methods of the invention. It will be apparent to those skilled in the art that substantial variations may be used in accordance with specific requirements. For example, customized hardware might also be used and/or particular elements might be implemented in hardware, software (including portable software, such as applets), or both. Further, connection to other computing devices such as network input/output devices may be employed.

As an example of one of the embodiments of the present invention, the multispectral conditions may comprise a plurality of different wavelengths of illumination light. The texture measure may be a measure of image contrast generated for certain spatial frequencies of the image. "Image contrast" refers herein to a measure of the tonal variation relative to the average tonal value over some region of an image. In the case of a living, uncovered human finger imaged using visible wavelengths by methods similar to those described herein, a significant portion of the fingerprint signal is due to capillary blood. This is due, in part, to the known physiological characteristic that the capillaries in the finger follow the pattern of the external fingerprint ridge structure. Therefore, the contrast of the fingerprint features relative to the illumination wavelength may be expected to be related to the spectral features of blood. In particular, the contrast of images taken with wavelengths longer than about 580 nm are significantly reduced relative to those images taken with wavelengths less than about 580 nm. Fingerprint patterns generated with nonblood pigments and other optical effects such as Fresnel reflectance have a different spectral contrast.

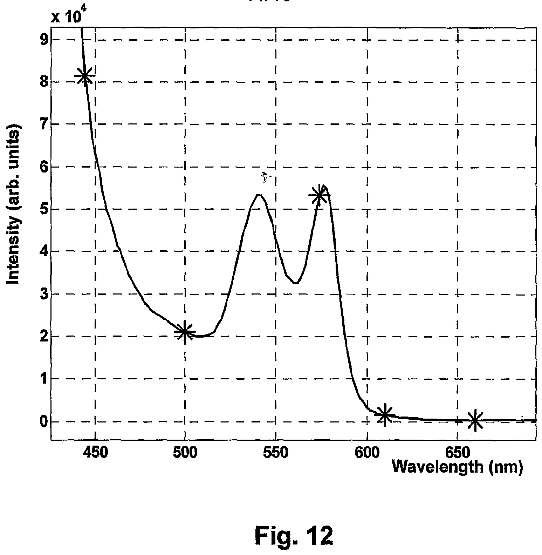

These absorbance spectrum of oxygenated blood over a wavelength range from about 425 nm to about 700 nm is shown in Fig. 12. In an exemplary embodiment, five different wavelengths are used to define the multispectral conditions, in this instance 445 nm, 500 nm, 574 nm, 610 nm, and 660 nm, although the invention is not limited to the use of any particular wavelengths or number of multispectral conditions used. Each of these wavelengths is identified in the spectrum of Fig. 12. As seen in the figure, the absorbance values of blood are largest for the exemplary wavelengths of 445 nm and 574 nm, less for 500 nm, and are very small for 610 nm and 660 nm. Therefore, images taken of an authentic finger under 445 nm and 574 nm illumination wavelengths may be expected to provide relatively high contrast and that images taken at 610-nm and 660-nm wavelengths provide relatively low contrast, with images taken at 500 nm having intermediate contrast. It is anticipated that the effects from other absorbers such as melanin, changes in scatter per wavelength, and various chromatic aberrations also affect the measures of contrast and/or other texture measures, but these effects are either small or also characteristic of a living human finger relative to other potential spoofing materials and methods.

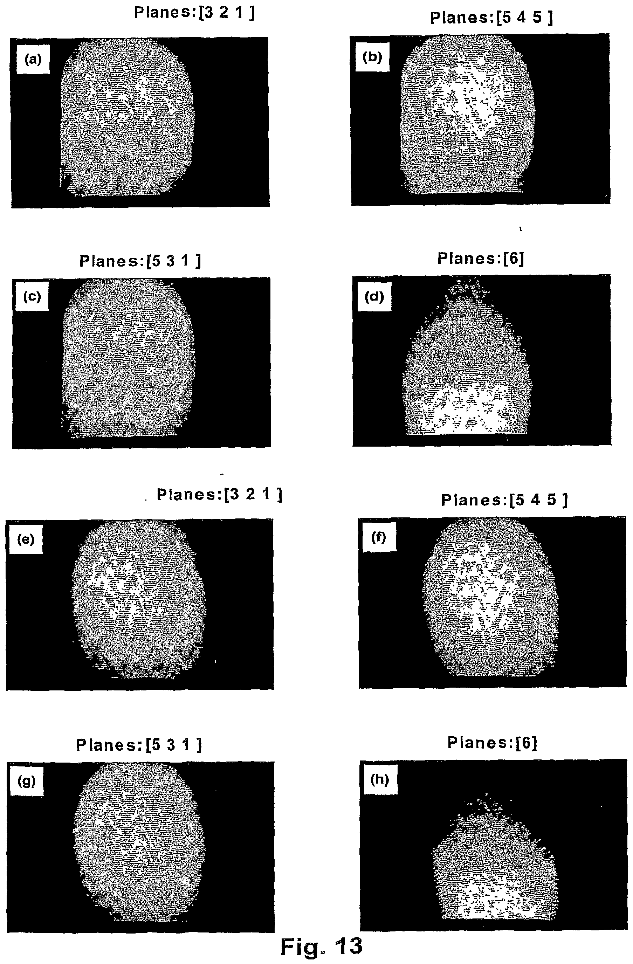

The various embodiments have been tested on data sets using twenty normal-finger images collected across thirteen people and using ten spoof images collected with thin silicone spoofs. Examples of images that may be collected are shown in Fig. 13, with parts (a) - (d) of that drawing showing results for real fingers and parts (e) - (h) showing results for spoofs. The "planes" identified for each of the images correspond to the different wavelengths, i.e. plane 1 is at 445 nm, plane 2 is at 500 nm, plane 3 is at 574 nm, plane 4 is at 610 nm, and plane 5 is at 660 nm. The origin of plane 6 can be understood with reference to Fig. 1B. Plane 6 corresponds to an image collected using the TIR illumination 153 but imaged by imaging system 159. The wavelength of the TIR illumination used in this experiment was approximately 640 nm. A comparison of the images may thus be made between real-finger-spoof pairs, i.e. between parts (a) and (e), which both show results for planes [3 2 1], where the notation [3 2 1] means that plane 3 has been mapped to red values in the color figure, plane 2 has been mapped to green values, and plane 1 has been mapped to blue values. Similarly, a comparison may be made between parts (b) and (f), which both show results for planes [5 4 5]; between parts (c) and (g), which both show results for planes [5 3 1]; and between parts (d) and (h), which both show TIR-illumination results. The [3 2 1] images of parts (a) and (e) are taken with blue and green illumination and show that for both the real tissue and the spoofs that the fingerprint ridges are relatively high contrast. This is opposite to the result for the [5 4 5] images of parts (b) and (f), which were taken under red illumination and show relatively low contrast for the fingerprint ridges of the real finger but relatively high contrast for the fingerprint ridges of the spoof.

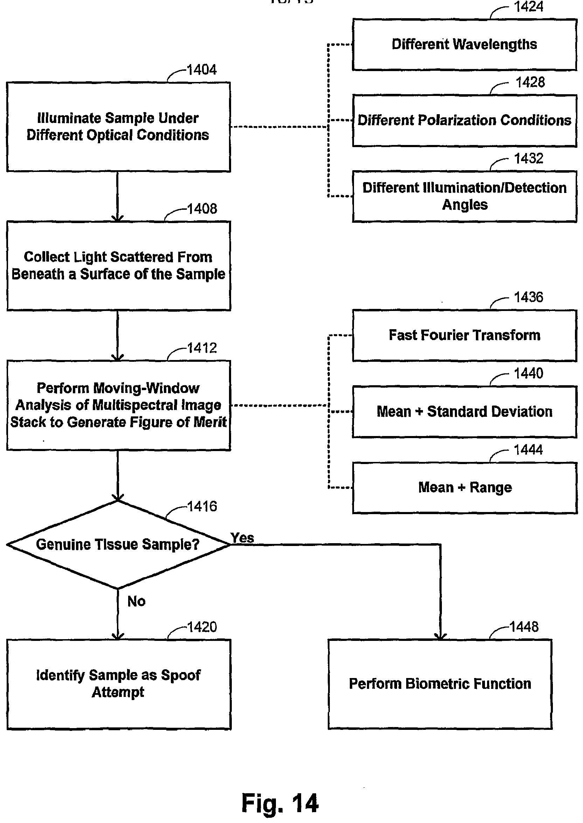

Exploiting such differences is achieved with methods of the invention, which are summarized with the flow diagram of Fig. 14. One or more measures of texture are developed for each optical condition, and the resulting set of texture measures is compared with the expected values from an authentic sample. At block 1404, a sample purported to be a tissue sample presented to enable a biometric function is illuminated under different optical conditions. Examples of the different optical conditions may include such factors as different wavelengths as indicated at block 1424, different polarization conditions as indicated at block 1428, different light-interaction angles as indicated at block 1432, and the like. Light scattered from the sample is collected at block 1408 and used in the comparative texture analyses. Different embodiments make use of a different form of moving-window analysis of the multispectral image stack derived from the collected light to generate a figure of merit at block 1412, and thereby evaluate the measure of texture or figure of merit. In some embodiments, the moving window operation 1412 may be replaced with a block-by-block or tiled analysis. In some embodiments, a single region of the image or the whole image may be analyzed at one time. The drawing shows three examples of contrast figures of merit that may be used in different embodiments, but this specific identification is not intended to be limiting since other contrast figures of merit as well as other texture measures may be used in alternative embodiments, as will be evident to those of skill in the art after reading this disclosure.

In one embodiment, fast-Fourier transforms are performed on the multispectral image stack as indicated at block 1436. An in-band contrast figure of merit C is generated in such embodiments as the ratio of the average or DC power to in-band power. Specifically, for optical condition i, the contrast figure of merit is

$$C_i \equiv \frac{\sum_\xi \sum_\eta \left|F_i(\xi,\eta)\right|^2\Big|_{R_{\text{low}}^2 < (\xi^2 + \eta^2) < R_{\text{high}}^2}}{\left|F_i(0,0)\right|^2}.$$

In this expression, $F_i(\xi,\eta)$ is the Fourier transform of the image $f_i(x,y)$ taken under illumination condition i, where $x$ and *y* are spatial coordinates for the image. The range defined by $R_{low}$ and $R_{high}$ represents a limit on spatial frequencies of interest for fingerprint features. For example, $R_{low}$ may be approximately 1.5 fringes/mm in one embodiment and $R_{high}$ may be 3.0 fringes/mm. In embodiments where the multispectral conditions are defined entirely by different illumination wavelengths, each state i represents one of those illumination wavelengths $\lambda$. In an alternative formulation, the contrast figure of merit may be defined as the ratio of the integrated power in two different spatial frequency bands. The equation shown above is a specific case where one of the bands comprises only the DC spatial frequency.

In another embodiment, moving-window means and moving-window standard deviations are calculated for the multispectral stack and used to generate the figure of merit, as indicated at block 1440. In this embodiment, for each optical condition i, the moving-window mean $\mu_i$ and the moving-window standard deviation $\sigma_i$ are calculated from the collected image $f_i(x, y)$. The moving windows for each calculation may be the same size and may conveniently be chosen to span on the order of 2 - 3 fingerprint ridges. Preferably, the window size is sufficiently large to remove the fingerprint features but sufficiently small to have background variations persist. The figure of merit $C_i$ in this embodiment is calculated as the ratio of the moving-window standard deviation to the moving-window mean:

$$C_i = \frac{\sigma_i}{\mu_i}.$$

In still another embodiment, a similar process is performed but a moving-window range (*i.e.,* max(image values) - min(image values)) is used instead of a moving-window standard deviation. This is indicated generally at block 1444. Thus, similar to the previous embodiment, a moving-window mean $\mu_i$ and a moving-window range $\delta_i$ are calculated from the collected image $f_i(x,y)$ for each optical condition i. The window size for calculation of the moving-window mean is again preferably large enough to remove the fingerprint features by small enough to maintain background variations. In some instances, the window size for calculation of the moving-window mean is the same as for calculation of the moving-window range, a suitable value in one embodiment spanning on the order of 2 - 3 fingerprint ridges. The figure of merit in this embodiment is calculated as the ratio of the moving-window mean:

$$C_i = \frac{\delta_i}{\mu_i}.$$

This embodiment and the preceding one may be considered to be specific cases of a more general embodiment in which moving-window calculations are performed on the multispectral stack to calculate a moving-window centrality measure and a moving-window variability measure. The specific embodiments illustrate cases in which the centrality measure comprises an unweighted mean, but may more generally comprise any other type of statistical centrality measure such as a weighted mean or median in certain embodiments. Similarly, the specific embodiments illustrate cases in which the variability measure comprises a standard deviation or a range, but may more generally comprise any other type of statistical variability measure such as a median absolute deviation or standard error of the mean in certain embodiments.

The results of the moving-window analysis are evaluated at block 1416 to determine whether they are consistent with the sample being a genuine tissue sample. If so, the biometric function may be performed at block 1448, usually including a further analysis to identify, or verify the identity of, the person to whom the tissue belongs. If the results are inconsistent with the sample being a genuine tissue sample, it is identified as a spoof at block 1420. Classification methods such as linear discriminant analysis, quadratic discriminant analysis, K nearest neighbors, support vector machines, decision trees, and other such methods as known to one of skill in the art may be employed to perform such identification.

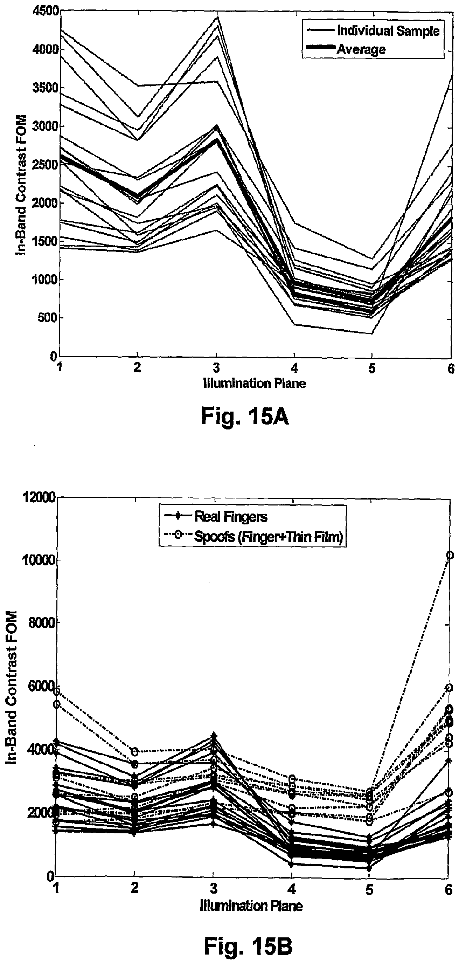

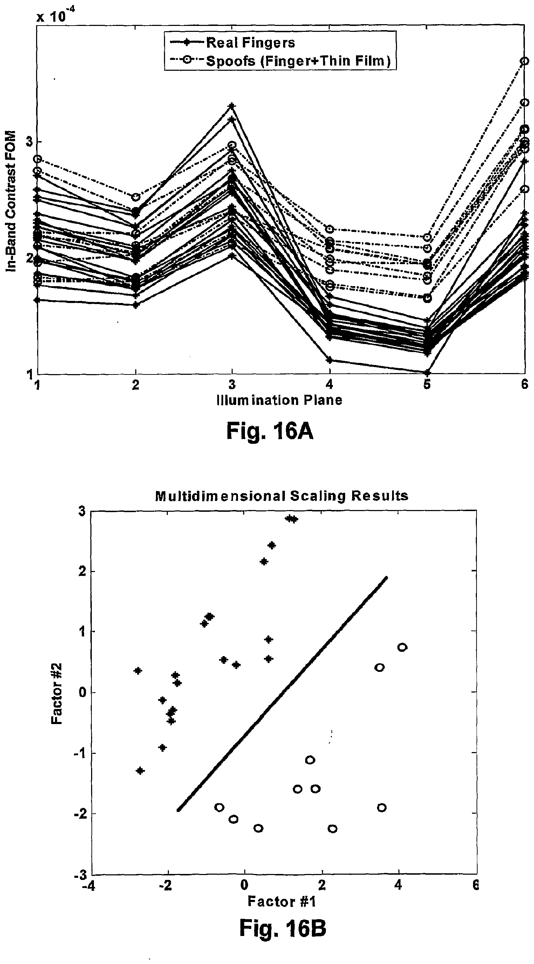

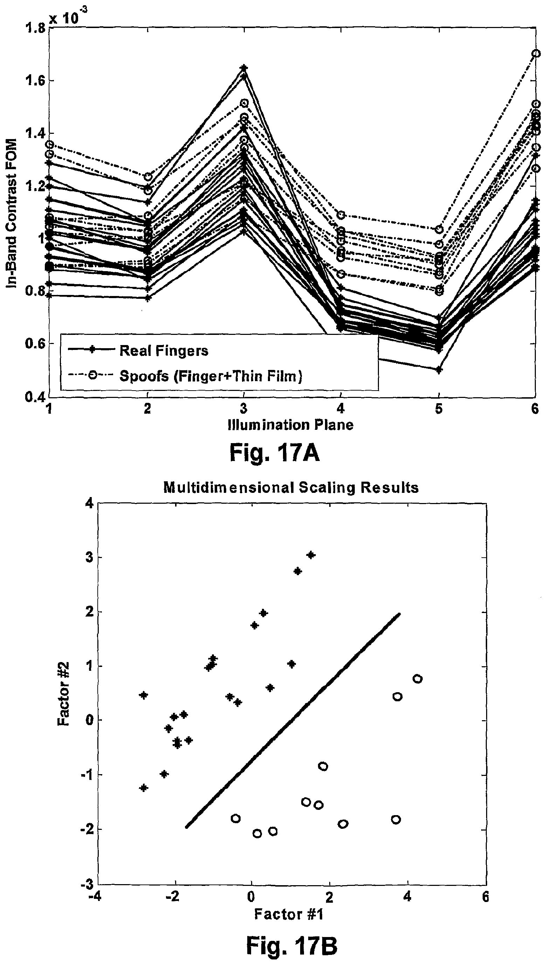

Exemplary results for each of the methods described above are provided in Figs. 15A - 17B. Figs. 15A - 15C provide results in which the figure of measure is calculated from moving-window fast Fourier transforms; Figs. 16A and 16B provide results in which the figure of measure is calculated from a moving-window mean and a moving-window standard deviation; and Figs. 17A and 17B provide results in which the figure of measure is calculated from a moving-window mean and a moving-window range.

The results shown in Fig. 15A were collected for real fingers and show results for the in-band contrast figure of merit derived from the moving-window fast Fourier transforms for each of multiple samples. The results for each sample are plotted along an abscissa identifying the illumination planes specified above; the abscissa may thus be considered to illustrate a wavelength dependence although the illumination plane numbers correspond somewhat nonlinearly to the wavelength. The data for individual samples are joined with gray lines and an average over all of the samples is shown with the thicker black line. Most of the results from real-finger samples show a characteristic and expected "V" shape in the blue-green region of the spectrum (planes 1 - 3) with a marked decrease of contrast at red wavelengths of planes 4 - 5, as expected due to the blood absorbance characteristics illustrated in Fig. 12. The contrast values for plane 6 are not consistent with the other planes due to the different optical geometry used to collect these images, but the values from this plane do provide additional discrimination ability.

Results for both real fingers and for spoofs are shown in Fig. 15B. In this instance, the results for the real fingers are shown with diamonds connected by solid lines while the results for the spoofs are shown with circles connected by dotted lines. The spoofs were real fingers covered by a thin film. As evident from an examination of the results at illumination planes 4, 5, and 6, the spoofs show higher contrast under red illumination than is anticipated for a genuine sample. In some instances, a relative comparison of the contrast figures of merit may be more robust than an absolute determination, such as by evaluating whether the red contrast values are less than some factor $\alpha$ of the blue-green values. Different embodiments may use different values of $\alpha$, with suitable values expected to be about $\alpha = 0.5$, $\alpha = 0.75$, etc.

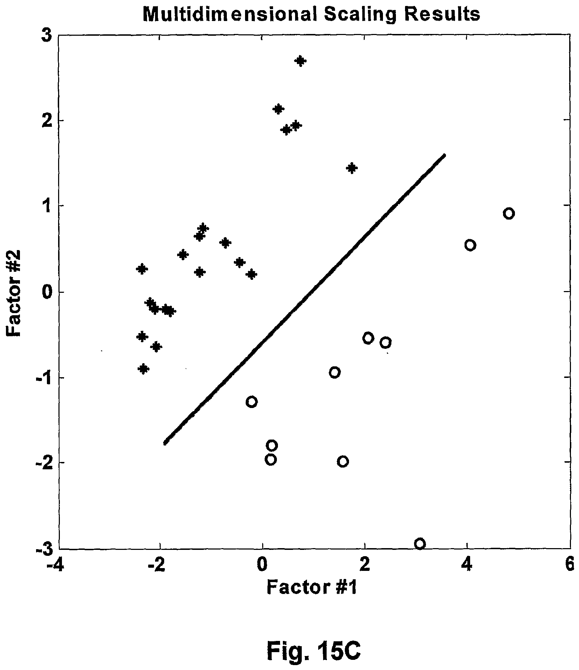

Multidimensional scaling may be used to process the data to provide a clear quantitative discriminator between real tissue and spoofs. This is illustrated in Fig. 15C, which shows multidimensional scaling results plotted for two coordinates. The coordinates in this instance correspond to scores of eigenvectors derived from a principal component analysis ("PCA") of the contrast data; specifically, "Factor 1" and "Factor 2" in this illustration are the scores for the first two eigenvectors generated from the data by the MatLab® program, available from The Mathworks, a company headquartered in Natick, Massachusetts. The derived results for the real fingers are shown with asterisks, while circles are used to denote the derived results for the spoofs. A clear division is evident between the two, permitting the results of Fig. 15C to be used as a separability plot - the line drawn in the graph is one potential demarcating line. An unknown sample analyzed in the same way that generates a result above and to the left of the line may be identified as a genuine sample at block 1416 of Fig. 14, while a result below and to the right of the line may be identified as a spoof. While the results here are shown for a two-dimensional space with different regions corresponding to different characterizations of the sample, more generally different regions of a space of any dimensionality may be used to discriminate the sample, such as by having hyperplanes demarcating the separation. More generally, as described elsewhere in this disclosure, a variety of different classification methods may be employed to classify the sample as an authentic finger or not.

The results of Fig. 16A are similar to those of Fig. 15B but were derived in an embodiment that uses a moving-window mean and a moving-window standard deviation as described above. Results from real fingers are again denoted with diamonds connected by solid lines while the results from spoofs are denoted with circles connected by dashed lines. Like the results of Fig. 15B, the spoofs show higher contrast under red illumination than a genuine sample. A similar separability plot to that of Fig. 15C is shown in Fig. 16B, derived using the results of Fig. 16A. In this instance, the results for real fingers are again denoted by asterisks while circles are used to denote the results for spoofs, and the results were again derived as eigenvector scores from a PCA, specifically using the first two eigenvectors provided by the MatLab® program. A demarcation line is shown that permits spoofs to be discriminated from real tissue by which portion of the two-dimensional space the multidimensional scaling results fall into. The general behavior shown in Fig. 16B is similar to that shown in Fig. 15C.

The results presented in Figs.7A and 17B also correspond to those of Figs. 15B and 15C or of Figs. 16A and 16B. In this instance, the results were derived in an embodiment that uses a moving-window mean and a moving-window range of the multispectral stack as described above. The spoof results denoted with circles connected by dashed lines again show higher contrast under red illumination that do the genuine-sample results denoted with diamonds connected by solid lines in Fig. 17A. The separability plot of Fig. 17B again shows the ability to define a demarcation line that separates the space of eigenvector scores into a region where real-tissue results (asterisks) are clearly separated from spoof results (circles). As previously noted, such a demarcation capability permits unknown results to the classified at block 1416 of Fig. 14 according to which portion of the multidimensional space the derived results fall.

Various alternative methods may also be used in different embodiments to evaluate the contrast data as shown in such drawings as Figs. 15A, 16A, and 17A. For example, linear discriminant analysis ("LDA") is used in one such alternative embodiments as a discriminator for contrast. Merely by way of example, such analysis may make use of training with two classes, a first class of contrast curves generated with human tissue and a second class of contrast curves generated with a variety of spoof samples.

While the various examples provided above focus on the use of fingers to provide tissue and on the use of different illumination-light wavelengths to provide multispectral conditions, this is not indented to be limited. In other embodiments, different polarization conditions may alternatively or additionally be used in defining the multispectral conditions. For example, comparisons may be mode between two or more conditions of polarization, which may include cross-polarized imaging, parallel-polarized imaging, random-polarized imaging, and the like. In addition, the incident light may have different polarization states, such as by having linearly polarized light, circularly polarized light, elliptically polarized light, or the like. In still other instances, different illumination angles may be used to provide multispectral conditions. The penetration depth of the light varies as a function of the illumination angle, resulting in changes in image contrast that may be analyzed as described above in discriminating between real tissue and spoofs.

Furthermore, the invention is not limited to the specific figures of merit described above and different or additional texture-based figures of merit may be used alternatively or additionally in various embodiments. For instance, statistical moments may be used, as may moment invariants, gray-level spatial dependencies, and the like in deriving figures of merit that discriminate samples as described.

Spectral contrast is a relative metric. An advantageous consequence of this fact is that the methods described above are expected to be robust across a variety of different types of spectral sensors and in a variety of different environmental conditions.

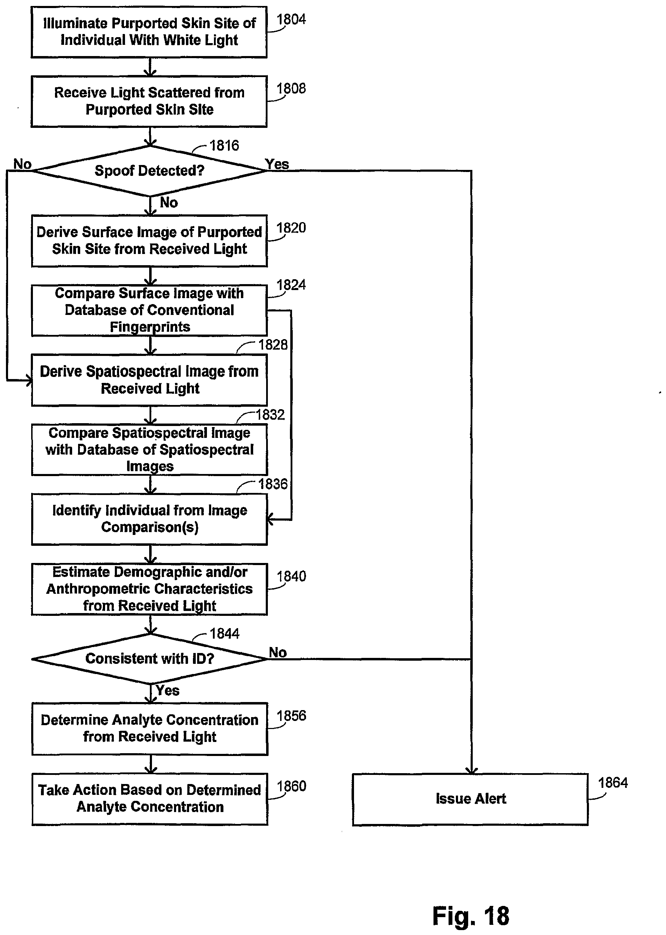

An overview of additional functionality that may be implemented with the computational device are summarized with the flow diagram of Fig. 18. In some embodiments, a purported skin site is illuminated as indicated at block 1804 with white light. This permits the biometric sensor to receive light from the purported skin site at block 1808. As described above, the received light may be analyzed in a number of different ways in implementing a biometric function. The flow diagram shows how certain combinations of analyses may be used in implementing the biometric function, although it is not necessary that all steps be performed. In other instances, a subset of the steps may be performed, additional steps might be performed, and/or the indicated steps might be performed in a different order than indicated.

At block 1812, a liveness check may be performed with the received light to confirm that the purported skin site is not some type of spoof, usually by verifying that it has the characteristics of living tissue. If a spool is detected, an alert may be issued at block 1864. The specific type of alert that is issued may depend on the environment in which the biometric sensor is deployed, with audible or visual alerts sometimes being issued near the sensor itself; in other instances, silent alerts may be transmitted to security or law-enforcement personnel.

The light received scattered from the purported skin site may be used at block 1820 to derive a surface image of the purported skin site. In instances where the purported skin site is a volar surface of a finger, such a surface image will include a representation of the pattern of ridges and valleys on the finger, permitting it to be compared with a database of conventional fingerprints at block 1824. In addition or alternatively, the received light may be used to derive a spatio-spectral image at block 1828. This image may be compared with a spatio-spectral database having images that are associated with individuals at block 1832. In either instance, the comparison may permit the individual to be identified at block 1836 as a result of the comparison. It is generally expected that higher-reliability identifications may be made by using the full spatio-spectral information to provide a comparison with spatiospectral images. But in some applications, there may be greater availability of conventional fingerprint data, with some individuals having their fingerprints stored in large law-enforcement fingerprint databases but not in spatio-spectral databases. In such cases, embodiments of the invention advantageously permit the extraction of a conventional fingerprint image to perform the identification.

The spatio-spectral data includes still additional information that may provide greater confidence in the identification, whether the identification is made by comparison with a conventional fingerprint database or through comparison with spatio-spectral information. For example, as indicated at block 1840, demographic and/or anthropometric characteristics may be estimated from the received light. When the database entry matched to the image at block 1836 includes demographic or anthropometric information, a consistency check may be performed at block 1844. For instance, an individual presenting himself may be identified as a white male having an age of 20 - 35 years from the estimated demographic and anthropometric characteristics. If the database entry against which the image is matched identifies the individual as a 68-year-old black woman, there is a clear inconsistency that would trigger the issuance of an alarm at block 1864.

Other information may also be determined from the received light, such as an analyte concentration at block 1856. Different actions may sometimes be taken in accordance with the determined analyte level. For example, ignition of an automobile might be prohibited if a blood-alcohol level exceeds some threshold, or an alarm might be issued if a blood-glucose level of a medical patient exceeds some threshold. Other physiological parameters, such as skin dryness conditions and the like, may be estimated in other applications, with still other actions sometimes being taken in response.

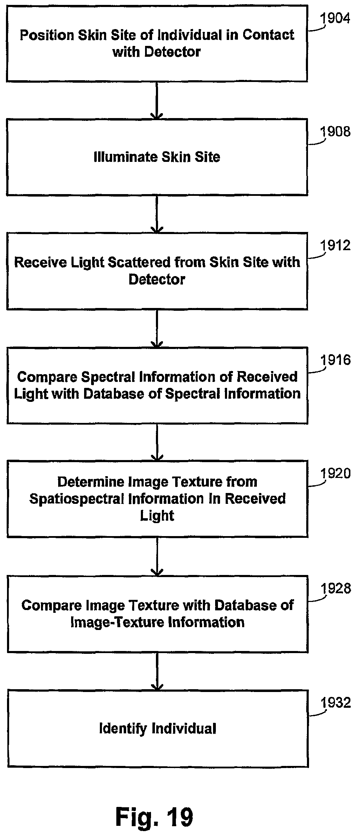

Fig. 19 provides a similar flow diagram to illustrate applications of a texture biometric sensor. The sensor is used by positioning a skin site of an individual in contact with the detector at block 1904. As previously noted, the detector may be relatively small so that only a portion of a finger surface is positioned in contact with the detector; because of the nature of texture biometrics, variations in the specific portion of the surface placed in contact during different measurements are not detrimental. Data are collected by illuminating the skin site at block 1908 and receiving light scattered from the skin site with the detector at block 1912.

The flow diagram indicates that different types of analyses may be performed. It is not necessary that each of these analyses be performed in every case and, indeed, it is generally expected that in most applications only a single type of analysis will be used. One category of analysis, indicated generally at block 1916, uses purely spectral comparisons of information. Another category of analysis, indicated generally at blocks 1920 and 1928 uses image texture information by determining the image texture from spatio-spectral information in the received light at block 1920 and comparing that image texture with a database of texture biometric information at block 1928. With either or both types of analysis, a biometric function is performed, such as identification of the individual at block 1932.

Thus, having described several embodiments, it will be recognized by those of skill in the art that various modifications, alternative constructions, and equivalents may be used without departing from the scope of the claims.

Accordingly, the above description should not be taken as limiting the scope of the invention, which is defined in the following claims.

**Claims**

1. An apparatus for evaluating genuineness of a sample presented for biometric evaluation, the apparatus comprising:

means (121; 323; 621; 710; 823; 1004) for illuminating the sample with light having a plurality of different wavelengths;
means (123; 325; 623; 720; 825; 1008) for receiving light scattered from the sample for each of the plurality of distinct optical conditions;
means (1102) for forming a plurality of images, each such image being formed from the received light for a respective one of the plurality of distinct optical conditions;
means (1102) for generating a plurality of figures of merit, each such figures of merit being generated from a respective one of the plurality of images; and
means (1102) for determining whether the generated plurality of figures of merit is consistent with the sample being authentic unconcealed biological tissue;

**characterised by**:

each figure of merit $C_i$ being determined using the equation:

$$C_i \equiv \frac{\sum_{\xi}\sum_{\eta}\left|F_i(\xi,\eta)\right|^2 \Big|_{R_{low}^2 < (\xi^2 + \eta^2) < R_{high}^2}}{\left|F_i(0,0)\right|^2}$$

wherein $F_i(\xi,\eta)$ is a Fourier transform of a corresponding one of the plurality of images $f_i(x,y)$ at a wavelength corresponding to index i, where x and y are spatial coordinates for the corresponding one of the plurality of images, and $R_{low}$ and $R_{high}$ each represent a limit on spatial frequencies of interest;
OR
each figure of merit $C_i$ being determined using the equation:

$$C_i = \frac{\sigma_i}{\mu_i}$$

wherein $\sigma_i$ is a moving-window standard deviation and $\mu_i$ is a moving-window mean, each calculated from a corresponding one of the plurality of images at a wavelength corresponding to index $i$;
OR
each a figure of merit $C_i$ being determined using the equation:

$$C_i = \frac{\delta_i}{\mu_i}$$

wherein $\delta_i$ is a moving-window range and $\mu_i$ is a moving-window mean, each calculated from a corresponding one of the plurality of images at a wavelength corresponding to index i.

2. The apparatus recited in claim 1 wherein the means (121; 323; 621; 823) for illuminating the sample with light having a plurality of different wavelengths further comprises means (107; 307; 607; 807) for illuminating the sample with light under a plurality of distinct polarization conditions.

3. The apparatus recited in claim 1 wherein the means (121; 323; 621; 710; 823) for illuminating the sample with light having a plurality of different wavelengths further comprises means for illuminating the sample with light at a plurality of distinct illumination angles.

4. The apparatus recited in claim 1 wherein the means (1102) for determining whether the generated plurality of figures of merit is consistent with the sample being authentic unconcealed biological tissue comprises means for confirming that an image contrast under red illumination is less than an image contrast under blue illumination.

5. The apparatus recited in claim 1 wherein the means (1102) for determining whether the generated plurality of figures of merit is consistent with the sample being authentic unconcealed biological tissue comprises means for confirming that an image contrast under red illumination is less than a predetermined value.

6. The apparatus recited in claim 1 wherein:

the means (1102) for generating a plurality of figures of merit further comprises means for applying a multidimensional scaling to map the plurality of figures of to a point in a multidimensional space; and
means (1102) for determining whether the generated plurality of figures of merit is consistent with the sample being authentic unconcealed biological tissue comprises means for determining whether the point is in a predefined region of the multidimensional space.

7. The apparatus recited in claim 6 wherein the multidimensional space is a two-dimensional space.

**Patentansprüche**

1. Vorrichtung zur Bewertung der Echtheit einer zur biometrischen Auswertung vorgelegten Probe, wobei die Vorrichtung Folgendes aufweist:

eine Einrichtung (121; 323; 621; 710; 823; 1004) zum Beleuchten der Probe mit Licht mit mehreren verschiedenen Wellenlängen;
eine Einrichtung (123; 325; 623; 720; 825; 1008) zum Empfangen eines von der Probe gestreuten Lichts für jede der mehreren unterschiedlichen optischen Bedingungen;
eine Einrichtung (1102) zum Erstellen von mehreren Bildern, wobei jedes derartige Bild aus dem empfangenen Licht für eine jeweilige Bedingung der mehreren unterschiedlichen optischen Bedingungen erstellt wird;
eine Einrichtung (1102) zum Erzeugen mehrerer Gütezahlen, wobei jede derartige Gütezahl aus einem jeweiligen Bild der mehreren Bilder erzeugt wird; und
eine Einrichtung (1102) zum Bestimmen, ob die erzeugten mehreren Gütezahlen mit der Probe konsistent sind, bei der es sich um authentisches, nicht verborgenes biologisches Gewebe handelt;
**dadurch gekennzeichnet, dass**:

jede Gütezahl $C_i$ unter Verwendung folgender Gleichung bestimmt wird:

$$C_i \equiv \frac{\sum_\xi \sum_\eta \left|F_i(\xi,\eta)\right|^2 \Big|_{R_{low}^2 < (\xi^2 + \eta^2) < R_{high}^2}}{\left|F_i(0,0)\right|^2}$$

worin $F_i(\xi, \eta)$ eine Fourier-Transformation eines entsprechenden Bildes der mehreren Bilder $f_i(x, y)$ bei einer dem Index i entsprechenden Wellenlänge ist, wobei $x$ und $y$ Raumkoordinaten für das entsprechende Bild der mehreren Bilder sind und $R_{low}$ und $R_{high}$ jeweils eine Grenze für interessierende Raumfrequenzen darstellen;
oder
wobei jede Gütezahl $C_i$ unter Verwendung folgender Gleichung bestimmt wird:

$$C_i = \frac{\sigma_i}{\mu_i}$$

worin $\sigma_i$ eine Standardabweichung des bewegten Fensters und $\mu_i$ ein Mittelwert des bewegten Fensters ist, die jeweils aus einem entsprechenden Bild der mehreren Bilder bei einer dem Index i entsprechenden Wellenlänge berechnet werden;
oder

wobei jede Gütezahl $C_i$ unter Verwendung folgender Gleichung bestimmt wird:

$$C_i = \frac{\delta_i}{\mu_i}$$

worin $\delta_i$ ein Bereich des bewegten Fensters und $\mu_i$ ein Mittelwert des bewegten Fensters ist, die jeweils aus einem entsprechenden Bild der mehreren Bilder bei einer dem Index i entsprechenden Wellenlänge berechnet werden.

2. Vorrichtung nach Anspruch 1, wobei die Einrichtung (121; 323; 621; 823) zum Beleuchten der Probe mit Licht mit mehreren verschiedenen Wellenlängen darüber hinaus eine Einrichtung (107; 307; 607; 807) zum Beleuchten der Probe mit Licht unter mehreren unterschiedlichen Polarisationsbedingungen aufweist.

3. Vorrichtung nach Anspruch 1, wobei die Einrichtung (121; 323; 621; 710; 823) zum Beleuchten der Probe mit Licht mit mehreren verschiedenen Wellenlängen darüber hinaus eine Einrichtung zum Beleuchten der Probe mit Licht bei mehreren unterschiedlichen Beleuchtungswinkeln aufweist.

4. Vorrichtung nach Anspruch 1, wobei die Einrichtung (1102) zum Bestimmen, ob die erzeugten mehreren Gütezahlen mit der Probe konsistent sind, bei der es sich um authentisches, nicht verborgenes biologisches Gewebe handelt, eine Einrichtung zum Bestätigen aufweist, dass ein Bildkontrast unter roter Beleuchtung geringer ist als ein Bildkontrast unter blauer Beleuchtung.

5. Vorrichtung nach Anspruch 1, wobei die Einrichtung (1102) zum Bestimmen, ob die erzeugten mehreren Gütezahlen mit der Probe konsistent sind, bei der es sich um authentisches, nicht verborgenes biologisches Gewebe handelt, eine Einrichtung zum Bestätigen aufweist, dass ein Bildkontrast unter roter Beleuchtung geringer ist als ein vorbestimmter Wert.

6. Vorrichtung nach Anspruch 1, wobei:

die Einrichtung (1102) zum Erzeugen mehrerer Gütezahlen darüber hinaus eine Einrichtung zum Anwenden einer mehrdimensionalen Skalierung aufweist, um die mehreren Zahlen auf einen Punkt in einem mehrdimensionalen Raum abzubilden; und
die Einrichtung (1102) zum Bestimmen, ob die erzeugten mehreren Gütezahlen mit der Probe konsistent sind, bei der es sich um authentisches, nicht verborgenes biologisches Gewebe handelt, eine Einrichtung zum Bestimmen aufweist, ob der Punkt in einem vordefinierten Bereich des mehrdimensionalen Raums liegt.

7. Vorrichtung nach Anspruch 6, wobei der mehrdimensionale Raum ein zweidimensionaler Raum ist.

**Revendications**

1. Appareil pour évaluer l'authenticité d'un échantillon présenté pour une évaluation biométrique, l'appareil comprenant :

des moyens (121 ; 323 ; 621 ; 710 ; 823 ; 1004) pour éclairer l'échantillon avec de la lumière ayant une pluralité de longueurs d'onde différentes ;

des moyens (123 ; 325 ; 623 ; 720 ; 825 ; 1008) pour recevoir la lumière diffusée en provenance de l'échantillon pour chaque état parmi la pluralité d'états optiques distincts ;

des moyens (1102) pour former une pluralité d'images, chaque image étant formée à partir de la lumière reçue pour un état respectif parmi la pluralité d'états optiques distincts ;

des moyens (1102) pour générer une pluralité des figures de mérite, chaque figure de mérite étant générée à partir d'une image respective parmi la pluralité d'images ; et

des moyens (1102) pour déterminer si oui ou non la pluralité générée de figures de mérite est cohérente avec l'échantillon de tissu biologique non dissimulé authentique ;

**caractérisé par** :

chaque figure de mérite $C_i$ étant déterminée au moyen de l'équation suivante :

$$C_i \equiv \frac{\sum_{\xi} \sum_{\eta} |F_i(\xi, \eta)|^2 \Big|_{R_{low}^2 < (\xi^2 + \eta^2) < R_{high}^2}}{|F_i(0,0)|^2}$$

dans lequel $F_i(\xi, \eta)$ est une transformation de Fournier d'une image correspondante parmi la pluralité d'images $f_i(x,y)$ à une longueur d'onde correspondant à l'indice $i$, dans lequel $x$ et $y$ sont des coordonnées spatiales pour l'image correspondante parmi la pluralité d'images, et $R_{low}$ et $R_{high}$ représentent chacun une limite sur des fréquences spatiales d'intérêt ;

OU

chaque figure de mérite $C_i$ étant déterminée au moyen de l'équation :

$$C_i = \frac{\sigma_i}{\mu_i}$$

dans lequel $\sigma_i$ est une déviation standard de fenêtre mobile et $\mu_i$ est un moyen de fenêtre mobile, chacun étant calculé à partir d'une image correspondante parmi la pluralité d'images à une longueur d'onde correspondant à l'indice $i$ ;

OU

chaque figure de mérite $C_i$ étant déterminée au moyen de l'équation :

$$C_i = \frac{\delta_i}{\mu_i}$$

dans lequel $\delta_i$ est une plage de fenêtre mobile et $\mu_i$ est un moyen de fenêtre mobile, chacun étant calculé à partir d'une image correspondante parmi la pluralité d'images à une longueur d'onde correspondant à l'indice i.

2. Appareil selon la revendication 1, dans lequel les moyens (121 ; 323 ; 621 ; 823) pour éclairer l'échantillon avec de la lumière ayant une pluralité de longueurs d'onde différentes comprend en outre des moyens (107 ; 307 ; 607 ; 807) pour éclairer l'échantillon avec de la lumière dans une pluralité d'états de polarisation distincts.

3. Appareil selon la revendication 1, dans lequel les moyens (121 ; 323 ; 621 ; 710 ; 823) pour éclairer l'échantillon avec de la lumière ayant une pluralité de longueurs d'onde différentes comprend en outre des moyens pour éclairer l'échantillon avec de la lumière sous une pluralité d'angles d'éclairage distincts.

4. Appareil selon la revendication 1, dans lequel les moyens (1102) pour déterminer si oui ou non la pluralité générée de figures de mérite est cohérente avec l'échantillon sous forme forme de tissu biologique non dissimulé authentique comprend des moyens pour confirmer qu'un contraste d'image sous éclairage rouge est inférieur à un contraste d'image sous éclairage bleu.

5. Appareil selon la revendication 1, dans lequel les moyens (1102) pour déterminer si oui ou non la pluralité générée de figures de mérite est cohérente avec l'échantillon sous forme de tissu biologique non dissimulé authentique comprend des moyens pour confirmer qu'une image contraste sous éclairage rouge est inférieure à une valeur prédéterminée.

6. Appareil selon la revendication 1, dans lequel :

les moyens (1102) pour générer une pluralité des figures de mérite comprennent en outre des moyens pour appliquer un échelonnement pluridimensionnel pour cartographier la pluralité des figures jusqu'au point dans un espace pluridimensionnel ; et
des moyens (1102) pour déterminer si oui ou non la pluralité générée de figures de mérite est cohérente avec l'échantillon sous forme de tissu biologique non dissimulé authentique comprend des moyens de détermination de si oui ou non le point est dans une région prédéfinie de l'espace pluridimensionnel.

7. Appareil selon la revendication 6, dans lequel l'espace pluridimensionnel est un espace bidimensionnel.

**Fig. 1**

Fig. 2A

Fig. 2B

**Fig. 3**

**Fig. 4**

**Fig. 5**

**Fig. 6**

## Fig. 7A

## Fig. 7B

**Fig. 8**

**Fig. 9**

EP 1 920 597 B1

1000

1008

1008

**Fig. 10A**

119

119

1000

1012

1000'

**Fig. 10B**

**Fig. 10C**

**Fig. 11**

**Fig. 12**

Fig. 13

┌─────────────────────────────┐
│ ┌─ 1424                      │
│ Different Wavelengths        │
└─────────────────────────────┘

┌─────────────────────────────┐
│ ┌─ 1404                      │
│ Illuminate Sample Under      │
│ Different Optical Conditions │
└─────────────────────────────┘

┌─────────────────────────────┐
│ ┌─ 1428                      │
│ Different Polarization Conditions │
└─────────────────────────────┘

┌─────────────────────────────┐
│ ┌─ 1432                      │
│ Different Illumination/Detection │
│ Angles                       │
└─────────────────────────────┘

┌─────────────────────────────┐
│ ┌─ 1408                      │
│ Collect Light Scattered From │
│ Beneath a Surface of the Sample │
└─────────────────────────────┘

┌─────────────────────────────┐
│ ┌─ 1436                      │
│ Fast Fourier Transform       │
└─────────────────────────────┘

┌─────────────────────────────┐
│ ┌─ 1412                      │
│ Perform Moving-Window        │
│ Analysis of Multispectral Image │
│ Stack to Generate Figure of Merit │
└─────────────────────────────┘

┌─────────────────────────────┐
│ ┌─ 1440                      │
│ Mean + Standard Deviation    │
└─────────────────────────────┘

┌─────────────────────────────┐
│ ┌─ 1444                      │
│ Mean + Range                 │
└─────────────────────────────┘

◇ 1416
Genuine Tissue Sample?   Yes

No

┌─────────────────────────────┐
│ ┌─ 1420                      │
│ Identify Sample as Spoof     │
│ Attempt                      │
└─────────────────────────────┘

┌─────────────────────────────┐
│ ┌─ 1448                      │
│ Perform Biometric Function   │
└─────────────────────────────┘

**Fig. 14**

**Fig. 15A**

**Fig. 15B**

Fig. 15C

## Fig. 16A

## Fig. 16B

Fig. 17A

Multidimensional Scaling Results

Fig. 17B

| Illuminate Purported Skin Site of Individual With White Light | 1804 |

| Receive Light Scattered from Purported Skin Site | 1808 |

1816
Spoof Detected?   No ... Yes
No

| Derive Surface Image of Purported Skin Site from Received Light | 1820 |

| Compare Surface Image with Database of Conventional Fingerprints | 1824 |

| Derive Spatiospectral Image from Received Light | 1828 |

| Compare Spatiospectral Image with Database of Spatiospectral Images | 1832 |

| Identify Individual from Image Comparison(s) | 1836 |

| Estimate Demographic and/or Anthropometric Characteristics from Received Light | 1840 |

1844
Consistent with ID?   No
Yes

| Determine Analyte Concentration from Received Light | 1856 |

| Take Action Based on Determined Analyte Concentration | 1860 |

| Issue Alert | 1864 |

Fig. 18

44

```
┌────────────────────────────────┐
│ Position Skin Site of Individual│   1904
│      in Contact with Detector   │
└────────────────────────────────┘
              │
              ▼
┌────────────────────────────────┐
│       Illuminate Skin Site      │   1908
└────────────────────────────────┘
              │
              ▼
┌────────────────────────────────┐
│ Receive Light Scattered from    │   1912
│      Skin Site with Detector    │
└────────────────────────────────┘
              │
              ▼
┌────────────────────────────────┐
│ Compare Spectral Information of │   1916
│ Received Light with Database of │
│      Spectral Information        │
└────────────────────────────────┘
              │
              ▼
┌────────────────────────────────┐
│   Determine Image Texture from  │   1920
│ Spatiospectral Information In    │
│        Received Light            │
└────────────────────────────────┘
              │
              ▼
┌────────────────────────────────┐
│ Compare Image Texture with      │   1928
│  Database of Image-Texture      │
│         Information             │
└────────────────────────────────┘
              │
              ▼
┌────────────────────────────────┐
│        Identify Individual       │   1932
└────────────────────────────────┘
```

**Fig. 19**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005059805 A2
- WO 2004090786 A2
- US 2002183624 A1
- US 37994506
- US 81869804, Robert K. Rowe
- US 17781705, Robert K. Rowe
- US 57636404, Robert K. Rowe and Stephen P. Corcoran
- US 60086704, Robert K. Rowe
- US 11510005, Robert K. Rowe

- US 11510105
- US 11507505
- US 65902405, Robert K. Rowe
- US 67577605, Robert K. Rowe
- US 015732
- US 6560352 B
- US 6816605 B
- US 6628809 B
- US 66088403, Robert K. Rowe
- US 87474001, Robert K. Rowe